(19) Europäisches Patentamt
European Patent Office
Office européen des brevets

(11) **EP 4 567 684 A1**

(12) **EUROPEAN PATENT APPLICATION**

(43) Date of publication:
**11.06.2025 Bulletin 2025/24**

(21) Application number: **23307166.1**

(22) Date of filing: **08.12.2023**

(51) International Patent Classification (IPC):
**G06N 10/20** (2022.01)   **G06N 10/40** (2022.01)
**G06N 10/60** (2022.01)   **G16C 10/00** (2019.01)

(52) Cooperative Patent Classification (CPC):
**G06N 10/60; G06N 10/20; G06N 10/40; G16C 10/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **BULL SAS**
  **78340 Les Clayes-Sous-Bois (FR)**
• **Electricité de France**
  **75008 Paris (FR)**
• **Pasqal**
  **91300 Massy (FR)**

(72) Inventors:
• **AYRAL, Thomas**
  **78000 VERSAILLES (FR)**
• **DOMAIN, Christophe**
  **92330 SCEAUX (FR)**
• **MICHEL, Antoine**
  **93160 NOISY LE GRAND (FR)**
• **HENRIET, Loïc**
  **91300 MASSY (FR)**

(74) Representative: **Plasseraud IP**
  **104 Rue de Richelieu**
  **CS92104**
  **75080 Paris Cedex 02 (FR)**

(54) **METHOD FOR DETERMINING A QUANTUM PHASE PARAMETER AND APPARATUS FOR IMPLEMENTING THE SAME**

(57)   A method for determining a quantum phase parameter characterizing a property of a quantum phase of a solid-state material sample, the method comprising: obtaining an estimate of a Hamiltonian operator $H$ of a lattice model of the solid-state material sample; mapping the estimate of the Hamiltonian operator $H$ onto a combination of a plurality of Hamiltonian operators comprising a Hamiltonian operator describing a longitudinal spin interaction and a transverse field; computing, using a quantum processor, an estimate of a property of the Hamiltonian operator describing a longitudinal spin interaction and a transverse field; computing an estimate of the quantum phase parameter as a property of the Hamiltonian operator $H$, based on the estimate of the property of the Hamiltonian operator describing a longitudinal spin interaction and a transverse field.

FIG. 2

**Description**

The present subject disclosure relates to the field of quantum computing, in particular to the determination of a parameter characterizing a quantum phase of a solid-state material using a quantum computer, and a quantum computer configured for performing such determination.

Quantum Computing is a field of computer science designed to solve some advanced problems faster than a classical computer, by using quantum mechanics. In this context, the term "classical" is often used to refer to a computer, a computing environment, a computing device (e.g. a processor) or a computing field which, as opposed to a reference to the term "quantum", does not use quantum mechanics.

The emerging field of Quantum Computing will have a significant impact in High Performance Computing (HPC), as HPC is already reaching the limit of classical computing. Consequently, HPC centers that only use classical computing may evolve to integrate quantum devices such as one or more Quantum Processing Units (QPUs) in the near future, to prepare for this new quantum revolution.

Strongly correlated electron systems display a large variety of exotic phases of matter, such as superconductivity, emerging from complex quantum many-body phenomena. While models of a Hamiltonian operator corresponding to such systems may be used to investigate properties thereof, solving such models may present computation complexity challenges when using a classical computer.

There is therefore a need for an improved method for determining a quantum phase parameter that addresses the drawbacks and shortcomings of the conventional technology in the art.

In particular, it is an object of the present subject disclosure to provide an improved method for determining a quantum phase parameter and apparatuses for implementing the same that address the drawbacks and shortcomings of the conventional technology in the art.

Another object of the present subject disclosure is to provide an improved method for determining a quantum phase parameter that characterizes a quantum phase of a solid-state material and apparatuses for implementing the same that address the drawbacks and shortcomings of the conventional technology in the art.

To achieve these objects and other advantages and in accordance with the purpose of the present subject disclosure, as embodied and broadly described herein, in one aspect of the present subject disclosure, a method for determining a quantum phase parameter, such as for example a quasi-particle weight, characterizing a property of a quantum phase of a solid-state material sample, is proposed. The proposed method may comprise one or more of the following: obtaining an estimate of a Hamiltonian operator $H$ of a lattice model of the solid-state material sample, mapping the estimate of the Hamiltonian operator $H$ onto a combination of a plurality of Hamiltonian operators comprising a Hamiltonian operator describing a longitudinal spin interaction and a transverse field, computing, using a quantum processor, an estimate of a property of the Hamiltonian operator describing a longitudinal spin interaction and a transverse field, and computing an estimate of the quantum phase parameter as a property of the Hamiltonian operator $H$, based on the estimate of the property of the Hamiltonian operator describing a longitudinal spin interaction and a transverse field.

The proposed scheme advantageously provides a method to determine a parameter characterizing a property of a quantum phase of a solid-state material which uses the computational power of quantum computing, thereby avoiding the computational challenges faced for such determination on a classical computer. The proposed therefore advantageously provides a method for determining properties (for example quantum phase properties) of solid-state material that were not reachable through computations performed on classical computers.

Embodiments of the proposed scheme may advantageously be used for investigating properties of quantum phases of any solid-state material, including a crystal, for using such solid-state material according to the properties determined thanks to the proposed scheme.

Advantageously, thanks to using a model such as a Hubbard model of the solid-state material, and processing such model according to the proposed scheme, materials with open shells, such as materials with d and f valence electrons, can be investigated for use in various use cases.

For instance, in some embodiments, the phase diagram of iron (Fe) and its alloys can only be understood by performing similar computations for certain phases. Better understanding properties of iron has many applications, such as for instance, but not limited to, understanding ageing phenomena in nuclear power plants and manufacturing nuclear power plant parts according to the determined properties.

In one or more embodiments, the plurality of Hamiltonian operators may further comprise a free fermionic Hamiltonian operator with renormalized hopping, and the proposed method may further comprise, the method further comprising: computing, using a non-quantum processor, an estimate of a property of the free fermionic Hamiltonian operator with renormalized hopping. In some embodiments, the estimate of the quantum phase parameter may be further based on the estimate of the property of the free fermionic Hamiltonian operator.

In one or more embodiments, the Hamiltonian operator describing a longitudinal spin interaction and a transverse field may comprise a transverse field Ising Hamiltonian operator.

In one or more embodiments, the Hamiltonian operator describing a longitudinal spin interaction and a transverse field may comprise a combination of a longitudinal spin interaction term and a transverse field term.

In one or more embodiments, the property may relate to a quasiparticle weight of the solid-state material sample.

In one or more embodiments, the parameter may characterize the behaviour of the solid-state material sample in an equilibrium state of the solid-state material sample.

In one or more embodiments, the parameter may characterize the behaviour of the solid-state material sample in an out of equilibrium state of the solid-state material sample.

In one or more embodiments, wherein obtaining an estimate of a Hamiltonian operator $H$ of a lattice model of the solid-state material sample may comprise: obtaining an estimate of a hopping parameter t for the solid-state material sample, and obtaining an estimate of an on-site interaction parameter $U$ for the solid-state material sample. In some embodiments, the obtaining the estimate of the hopping parameter t for the solid-state material sample may comprise: measuring the estimate of hopping parameter $t$ on the solid-state material sample. In some embodiments, the obtaining the estimate of the on-site interaction parameter $U$ for the solid-state material sample may comprise: measuring the estimate of the on-site interaction parameter $U$ on the solid-state material sample.

In one or more embodiments, the model may be a Fermi-Hubbard model describing the material as a lattice with interacting electrons.

In one or more embodiments, the model may be a single band model.

In one or more embodiments, the model may be a half-filled model.

In one or more embodiments, the quantum processor may be an analogue quantum processor.

In one or more embodiments, the quantum processor may be a Rydberg quantum processor.

In one or more embodiments, the proposed method may further comprise: solving the Hamiltonian operator of the Fermi-Hubbard model by performing one or more iterations of a model solving loop, an iteration of the model solving loop comprising:

Computing, using the non-quantum processor, a correlation function denoted $\langle f^+ f \rangle_{nn}$ of the transverse field Ising Hamiltonian operator based on an initial value of a renormalized hopping parameter $Q$, using the correlation function $\langle f^+ f \rangle_{nn}$ of the transverse field Ising Hamiltonian operator to compute, using the quantum processor, a correlation function denoted $\langle S^z S^z \rangle_{nn}$ of the free fermionic Hamiltonian operator, and computing a value of the renormalized hopping parameter $Q$ based on the correlation function $\langle S^z S^z \rangle_{nn}$.

According to another aspect of the present subject disclosure, an apparatus (e.g. a hybrid computer system comprising both classical and quantum computation resources) is proposed, which comprises a processor, and a memory operatively coupled to the processor. The proposed apparatus is configured to perform embodiments of a proposed method according to the present subject disclosure.

According to another aspect of the present subject disclosure, a quantum computer apparatus (e.g. configured for analogue quantum computing) is proposed, which comprises a quantum processor, and a memory operatively coupled to the processor. The proposed apparatus is configured to compute, using the quantum processor, an estimate of a property of a Hamiltonian operator describing a longitudinal spin interaction and a transverse field as part of performing embodiments of a proposed method according to the present subject disclosure.

According to yet another aspect of the present subject disclosure, a non-transitory computer-readable medium encoded with executable instructions which, when executed on a non-quantum computer, causes a processor of the non-quantum computer, that is operatively coupled with a memory, to perform embodiments of a proposed method according to the present subject disclosure, is proposed.

According to yet another aspect of the present subject disclosure, a computer program product comprising computer program code (tangibly) embodied in a computer readable medium, said computer program code comprising instructions to, when provided to a non-quantum computer system and executed, cause said computer to perform embodiments of a proposed method according to the present subject disclosure, is proposed.

According to yet another aspect of the present subject disclosure, a computer program product comprising computer program code (tangibly) embodied in a computer readable medium, said computer program code comprising instructions to, when provided to a quantum computer system and executed, cause said computer to compute, using a quantum processor of the quantum computer system, an estimate of a property of a Hamiltonian operator describing a longitudinal spin interaction and a transverse field as part of performing embodiments of a proposed method according to the present subject disclosure, is proposed.

It should be appreciated that the present invention can be implemented and utilized in numerous ways, including without limitation as a process, an apparatus, a system, a device, and as a method for applications now known and later developed. These and other unique features of the system disclosed herein will become more readily apparent from the following description and the accompanying drawings.

## Brief description of the drawings

The present subject disclosure will be better understood and its numerous objects and advantages will become more apparent to those skilled in the art by reference to the following drawings, in conjunction with the accompanying specification, in which:

Figure 1 shows an exemplary lattice with fermions that illustrates an exemplary lattice model of a solid-state material;

Figure 2 is a flow chart illustrating an exemplary method for determining a quantum phase parameter, in accordance with one or more embodiments;

Figure 3 illustrates an exemplary mapping of a Hubbard Hamiltonian modelled by the lattice of interacting electrons of Fig. 1 onto two Hamiltonians in accordance with one or more embodiments;

Figure 4 shows curves of estimated values of quasiparticle $Z$ as a function of the ratio $U/t$, for different numbers of sites in the lattice, in accordance with one or more embodiments;

Figures 5(a) - 5(d) show an exemplary dynamical response of a quasiparticle weight parameter after an interaction quench in accordance with one or more embodiments;

Figure 6 is a diagram illustrating an exemplary architecture of a computer system for implementing the proposed method, in accordance with one or more embodiments.

Figures 7 (a) and (b) show an exemplary schematic representation of a cluster geometry for (a) N = 4 sites and (b) 12 sites, in accordance with one or more embodiments.

Figure 8 (a) shows an exemplary Mott transition for an exemplary $3 \times 3$ cluster.

Figures 8 (b) to (d) illustrate an exemplary evolution of Z as a function of loop iterations for an exemplary 6 sites embedding, in accordance with one or more embodiments.

Figure 9 illustrates an exemplary impact of an imposed number of loops k in the slave-spin mean-field scheme for a cluster of N = 4 sites, in accordance with one or more embodiments.

Figures 10 (a) - (c) illustrate an exemplary optimization of geometry for an implementation on a quantum computer device, in accordance with one or more embodiments.

Figure 11 illustrates an exemplary outcome of equilibrium and out-of-equilibrium computations with a "perfect geometry" and an imperfect geometry, in accordance with one or more embodiments.

Figure 12 illustrates an exemplary effect of a total annealing time on the outcome of a simulation, in accordance with one or more embodiments.

Figure 13 illustrates an exemplary impact of switch-on time $\tau_{ramp}$ in a quench dynamic for a cluster of N = 4 sites, in accordance with one or more embodiments.

Figure 14 illustrates an exemplary effect of dephasing noise on the result for a cluster of N = 4 sites, in accordance with one or more embodiments.

Figure 15 illustrates an exemplary impact of measurement error for a cluster of N = 4 sites, in accordance with one or more embodiments.

Figure 16 illustrates an exemplary impact of different sampling rates $N_s$ on measured states for a cluster of N = 4 sites, in accordance with one or more embodiments.

## Description of embodiments

The advantages, and other features of the components disclosed herein, will become more readily apparent to those having ordinary skill in the art form. The following detailed description of certain preferred embodiments, taken in conjunction with the drawings, sets forth representative embodiments of the subject technology, wherein like reference numerals identify similar structural elements.

For simplicity and clarity of illustration, the drawing figures illustrate the general manner of construction, and descriptions and details of well-known features and techniques may be omitted to avoid unnecessarily obscuring the discussion of the described embodiments of the subject disclosure. Additionally, elements in the drawing figures are not necessarily drawn to scale. For example, the dimensions of some of the elements in the figures may be exaggerated relative to other elements to help improve understanding of embodiments of the present subject disclosure. Certain figures may be shown in an idealized fashion in order to aid understanding, such as when structures are shown having straight lines, sharp angles, and/or parallel planes or the like that under real-world conditions would likely be significantly less symmetric and orderly.

In addition, it should be apparent that the teaching herein can be embodied in a wide variety of forms and that any specific structure and/or function disclosed herein is merely representative. In particular, one skilled in the art will appreciate that an aspect disclosed herein can be implemented independently of any other aspects and that several aspects can be combined in various ways.

The present disclosure is described below with reference to functions, engines, block diagrams and flowchart illustrations of the methods, systems, and computer program according to one or more exemplary embodiments. Each described function, engine, block of the block diagrams and flowchart illustrations can be implemented in hardware, software, firmware, middleware, microcode, or any suitable combination thereof. If implemented in software, the functions, engines, blocks of the block diagrams and/or flowchart illustrations can be implemented by computer program instructions or software code, which may be stored or transmitted over a computer-readable medium, or loaded onto a general purpose non quantum computer, special purpose non quantum computer or other programmable data processing apparatus to produce a machine, such that the computer program instructions or software code which execute on the non quantum computer or other programmable data processing apparatus, create the means for implementing the functions described herein.

Embodiments of computer-readable media includes, but are not limited to, both computer storage media and communication media including any medium that facilitates transfer of a computer program from one place to another. As used herein, a "computer storage media" may be any physical media that can be accessed by a computer. Examples of computer storage media include, but are not limited to, a flash drive or other flash memory devices (e.g. memory keys, memory sticks, key drive), CD-ROM or other optical storage, DVD, magnetic disk storage or other magnetic storage devices, memory chip, RAM, ROM, EEPROM, smart cards, Solid State Drive (SSD) devices or Hard Disk Drive (HDD) devices, or any other suitable medium from that can be used to carry or store program code in the form of instructions or data structures which can be read by a computer processor. Also, various forms of computer-readable media may transmit or carry instructions to a computer, including a router, gateway, server, or other transmission device, wired (coaxial cable, fiber, twisted pair, DSL cable) or wireless (infrared, radio, cellular, microwave). The instructions may comprise code from any computer-programming language, including, but not limited to, assembly, C, C++, Visual Basic, HTML, PHP, Java, Javascript, Python, Julia, and bash scripting.

Unless specifically stated otherwise, it will be appreciated that throughout the following description discussions utilizing terms such as processing, computing, calculating, determining, generating, or the like, refer to the action or processes of a computer or computing system, or similar electronic computing device, that manipulate or transform data represented as physical, such as electronic, quantities within the registers or memories of the computing system into other data similarly represented as physical quantities within the memories, registers or other such information storage, transmission or display devices of the computing system.

The terms "comprise," "include," "have," and any variations thereof, are intended to cover a non-exclusive inclusion, such that a process, method, article, or apparatus that comprises a list of elements is not necessarily limited to those elements, but may include other elements not expressly listed or inherent to such process, method, article, or apparatus.

The terms "quantum" or "quantum computing" as used in the present subject disclosure are intended to cover any computer, computing system, processing or computing operation, configured to use or exploit quantum mechanical phenomena. A computer, processor, calculator, computing system, computing node, computing task, computer job, processing, algorithm, and processing resource configured to use or exploit quantum mechanical phenomena will be referred to herein as "quantum" (a quantum computer, quantum processor, quantum calculator, quantum computing system, quantum computing node, quantum computing task, quantum computer job, quantum processing, quantum algorithm, and quantum processing resource, respectively). In contrast, a computer, processor, calculator, computing system, computing node, computing task, computer job, processing, algorithm, and processing resource which is not configured to use or exploit quantum mechanical phenomena may be referred to herein as "classical" or "non-quantum" (a classical computer, classical processor, classical calculator, classical computing system, classical computing system, classical computing node, classical computing task, classical computer job, classical algorithm, classical processing, classical processing resource, respectively). A quantum processor (also referred to herein as a quantum processing unit (or QPU)) may be configured to perform both quantum processing and classical processing.

The terms "analog quantum computing" as used in the present subject disclosure refers to quantum computing that involves a continuous-time description of a computer program, as opposed to a logic gate-based (also referred to as "digital") description. Quantum operations are implemented through continuous operations, such as time-dependent laser-induced couplings or magnetic fields. The terms "analogue quantum processor", and "analogue quantum computer" respectively refer to a quantum processor and a quantum computer configured to perform analogue quantum computing.

The term "hybrid" as used in the present subject disclosure, for example as applied to a computer, an algorithm, a computer task, a computer job, refers to the combination of classical and quantum.

The terms "Rydberg atoms" as used in the present subject disclosure, for example as applied to a computer or a processor configured to use analogue quantum computing, refers to atoms trapped by optical tweezers and excited to high quantum numbers (which may sometimes be referred to as "Rydberg states").

Additionally, the word "exemplary" as used herein means serving as an example, instance, or illustration. Any aspect or design described herein as "exemplary" is not necessarily to be construed as preferred or advantageous over other aspects or designs.

The present subject disclosure may advantageously be implemented on any suitable computing environment comprising a quantum computer equipped with quantum processing resources (e.g. one or more QPUs, such as for example a Rydberg atoms processor). For example, the present subject disclosure may be implemented on a hybrid computer configured with one or more classical resources (e.g. one or more CPUs) and one or more quantum processing resources (e.g. one or more QPUs), such as a hybrid HPC cluster, an electronic component, an electronic chipset, a QPU, an electronic circuit-board, an electronic circuit, etc.

In the following, embodiments of the present subject disclosure are described in the exemplary case of a determination of a quantum phase parameter characterizing a property of a quantum phase of a crystal sample which relates to a quasiparticle weight of the crystal sample. However, a person of ordinary skill in the art would understand that the processes, apparatuses and computer programs of the present subject disclosure may be implemented for determining a quantum phase parameter characterizing any suitable property of any solid-state material sample, and that such proposed processes, apparatuses and computer programs of the present subject disclosure are not limited to the determination of a quantum phase parameter characterizing a property of a quantum phase of a crystal sample or to the determination of a quantum phase parameter characterizing a property of a quantum phase of a crystal sample related to a quasiparticle weight of the crystal sample, which is provided as an example only.

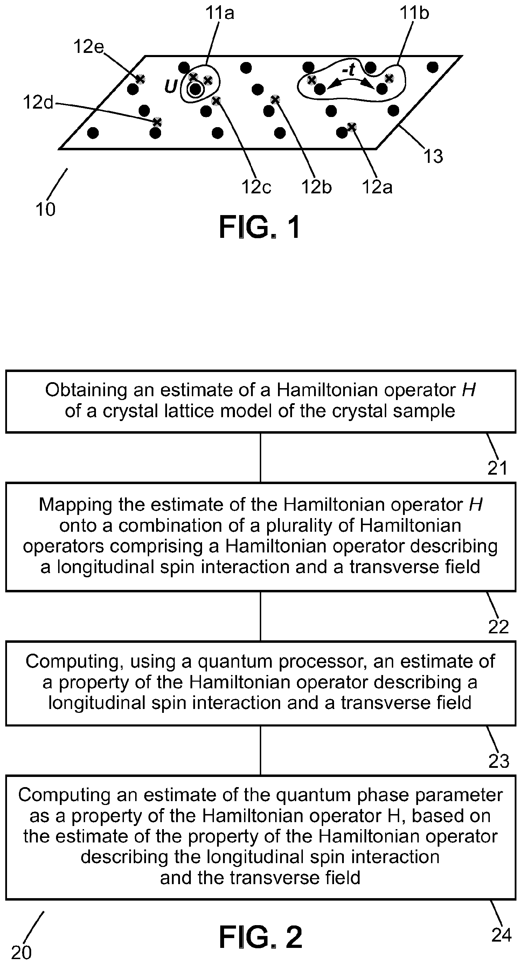

One may consider a lattice model (e.g. a crystal lattice model) of a solid-state material (e.g. a crystal sample) such as illustrated by the exemplary lattice with fermions (10) shown on Fig. 1.

Shown on Fig. 1 is an exemplary lattice (13) of a plurality of sites modeling atoms (atomic cores), on which electrons (fermionic particles, which may also be referred to herein as "fermions") (12a - 12e) may move from one site to another site of the lattice (13), thereby modelling a possibly conductive phase of the solid-state material in which fermions are moving in the solid-state material. Each of the fermions has a spin (denoted $\sigma$ in the following), and is subject to Coulomb interaction with each of the other fermions of the solid-state material, in particular those (11a) co-located on the same site of the lattice, while others (11b) are subject to hopping to one site to another.

A Hamiltonian operator may be used to describe the motion of the electrons of the solid-state material (sample), as in the field of quantum mechanics such operator is used to describe the energy of a quantum system (corresponding to the solid-state material sample).

Figure 2 shows a block diagram illustrating the determining (20) of a quantum phase parameter according to embodiments of the present subject disclosure.

One may consider solid-state material sample (e.g. a crystal sample) modeled by a lattice model, such as for example the lattice (10) of Fig. 1.

In one or more embodiments, an estimate of a Hamiltonian operator $H$ of the solid-state material lattice model of the solid-state material sample may be obtained (21).

In some embodiments, the solid-state material lattice model may be of the Fermi-Hubbard type which describes the solid-state material as a solid-state material lattice with interacting electrons.

In some embodiments, in order to reduce the complexity of the manipulated formal expressions, one or more of a single band model and a half-filled model may be used for implementing the proposed scheme.

A single band model may advantageously reduce the complexity of the manipulated formal expressions as it assumes that fermions of such model co-located on the same site can occupy only one type of molecular orbitals.

A half-filled model, which considers a single fermion per lattice site, may also advantageously reduce the complexity of the manipulated formal expressions as it will allow one to simplify the handling of the constraint introduced below.

In one or more embodiments, a single-band, half-filled Fermi-Hubbard model of the solid-state material sample on a square lattice may therefore be used.

In one or more embodiments, the estimate of the Hamiltonian operator $H$ may be mapped (22) onto a combination of a plurality of Hamiltonian operators comprising a Hamiltonian operator describing a longitudinal spin interaction and a transverse field.

The mapping of the estimate of the Hamiltonian operator $H$ onto a combination of a plurality of Hamiltonian operators may advantageously divide computations on the estimate of the Hamiltonian operator $H$ into computations on the plurality of Hamiltonian operators. Therefore, in some embodiments, computations on the Hamiltonian operator describing a longitudinal spin interaction and a transverse field, such as computation of an estimate of a property of such Hamiltonian operator, may be performed.

In some embodiments, an estimate of a property of the Hamiltonian operator describing a longitudinal spin interaction and a transverse field may advantageously be computed (23) directly on a quantum processor, thereby avoiding the challenge caused by tackling such computation on a non-quantum processor.

In some embodiments, an estimate of the quantum phase parameter of the solid-state material sample may be computed (24) as a property of the Hamiltonian operator $H$, based one the computed estimate of the property of the Hamiltonian operator describing a longitudinal spin interaction and a transverse field.

In one or more embodiments, the obtaining an estimate of a Hamiltonian operator $H$ of a crystal lattice model of the solid-state material sample may comprise: obtaining an estimate of a hopping parameter $t$ for the crystal sample, and obtaining an estimate of an on-site interaction parameter $U$ for the crystal sample.

In some embodiments, a Fermi-Hubbard Hamiltonian of the solid-state material sample model may be expressed as:

$$H_{Hubbard} = \sum_{i,j,\sigma} t_{ij} d_{i\sigma}^+ d_{j\sigma} + \frac{U}{2} \sum_i \left( n_i^d - 1 \right)^2$$

where $t_{ij}$ designates a hopping amplitude parameter describing the amplitude of hopping of a fermion hopping between a lattice site of index $i$ and another lattice site of index $j$, $d_{i\sigma}^+$ designates a creation operator that creates a fermion of spin $\sigma$ on a lattice site of index $i$, $d_{j\sigma}$ designates an annihilation operator that annihilates a fermion of spin $\sigma$ from a lattice site of index $j$, where $U$ designates an (a parameter related to) on-site Coulomb interaction between fermions, and $n_i^d$ designates (a parameter related to) the density operator of fermions at the site of index $i$.

In the above expression of the Hamiltonian $H_{Hubbard}$ of the solid-state material sample model (the quantum system), the infinite sum $\sum_{i,j,\sigma} t_{ij} d_{i\sigma}^+ d_{j\sigma}$ is a term which describes the kinetic energy of the quantum system modelled to contain an infinity of fermions (the solid-state material sample) (through kinetic energy of electrons hopping from a site of index i to a site of index j) and the infinite sum $\frac{U}{2} \sum_i \left( n_i^d - 1 \right)^2$ is a term which describes the potential energy of the quantum system (the solid-state material sample) resulting from the Coulomb electrostatic potential generated by the fermions (through the parameter $U$ describing on-site interactions between electrons).

In some embodiments, a Hamiltonian model limited to nearest-neighbor hopping may be used, which corresponds to an approximation which takes into account the probability of hop between two neighboring sites on the lattice which is higher than the probability of hop between two remote sites on the lattice.

For example, the proposed scheme may use a model in which $t_{ij} = -t\delta_{\langle ij \rangle}$, where $\delta_{\langle ij \rangle}$ is a Kronecker operator equal to 0 if lattice site $i$ and lattice site $j$ are not neighbor, and equal to 1 otherwise.

The above-stated estimate of the $H_{Hubbard}$ Hamiltonian model of strongly-correlated electrons is generically hard to solve on classical (non-quantum) computers, in particular with respect to out-of-equilibrium phases where the most advanced methods are usually limited to short-time dynamics.

In order to solve the estimate of Hamiltonian operator that was obtained, in one or more embodiments, the estimate of the Hamiltonian operator $H$ may be mapped onto a combination of a plurality of Hamiltonian operators comprising a Hamiltonian operator describing a longitudinal spin interaction and a transverse field.

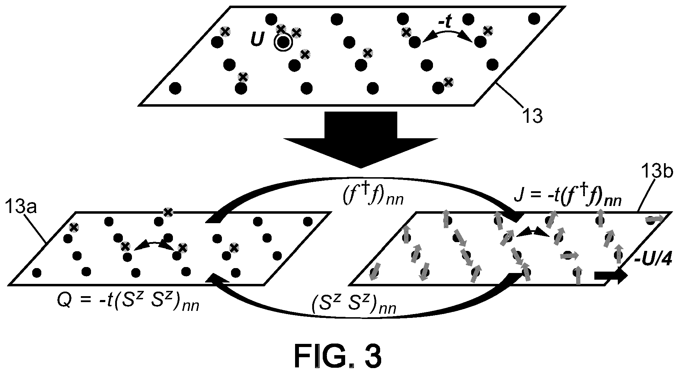

Figure 3 illustrates an exemplary mapping of a Hubbard Hamiltonian modelled by the lattice of interacting electrons of Fig. 1 onto two Hamiltonians.

Shown on Fig. 3 is an exemplary mapping of a Hubbard Hamiltonian modelled by the lattice of interacting electrons onto a plurality of Hamiltonians comprising a free fermionic Hamiltonian (13a) with renormalized hopping corresponding to the system energy from electrons hopping from one site to another, and a transverse field Ising Hamiltonian (13b) corresponding to the system energy from electrons subject to on-site interactions.

In some embodiments, the plurality of Hamiltonian operators further may therefore comprise, in addition to the Hamiltonian operator describing a longitudinal spin interaction and a transverse field, a free fermionic Hamiltonian operator with renormalized hopping.

Using such a mapping of the fermionic model of Hamiltonian operator advantageously avoids directly solving such model, and advantageously decomposes the solving problem using a separation of variables that singles out two important degrees of freedom of the model, namely spin and charge.

In one or more embodiments, the mapping of the Hamiltonian operator H onto a combination of a plurality of Hamiltonian operators may be determined by replacing the fermionic operator $d_{i\sigma}^+$ with a combination (for example the product) of a pseudo-fermion operator $f_{i\sigma}^+$ and an auxiliary spin field $S_i^z$ ($S_i^{a=x,y,z}$ denote the Pauli spin operators along the three axis $x$, $y$, and $z$). For example, in some embodiments, the fermionic operator $d_{i\sigma}^+$ may be replaced by an operator based on the product of the pseudo-fermion operator $f_{i\sigma}^+$ with the auxiliary spin field $S_i^z$ : $f_{i\sigma}^+ \cdot S_i^z$.

The ensuing enlargement of the Hilbert space is compensated for by imposing constraints such as

$$\frac{S_i^x + 1}{2} = \left(n_i^f - 1\right)^2$$

on each site. In the particle-hole symmetric case studied here, these constraints will be fulfilled automatically, as for example no projection to the physical portion of the Hilbert space will be needed, as opposed to the non-particle-hole-symmetric case, where constraints must be enforced.

In one or more embodiments, the combination of a plurality of Hamiltonian operators may comprise a Hamiltonian operator describing a longitudinal spin interaction and a transverse field $H_s(J)$.

In some embodiments, Hamiltonian operator describing a longitudinal spin interaction and a transverse field $H_s$ (J) may comprise a Ising transverse field Hamiltonian operator, such as the following Hamiltonian:

$$H_s(J) = \sum_{i,j} J_{ij} S_i^z S_j^z + \frac{U}{4} \sum_i S_i^x$$

where

$$J_{ij} = \sum_\sigma t_{ij} \langle f_{i,\sigma}^+ f_{j,\sigma} \rangle$$

and $\langle \ \rangle$ designates a statistic mean.

In some embodiments, the fermionic operator $d_{i\sigma}^+$ may be replaced by a product of the pseudo-fermion operator $f_{i\sigma}^+$ with the auxiliary spin field $S_i^z$, so the infinite sum $\sum_{i,j,\sigma} t_{ij} d_{i\sigma}^+ d_{j\sigma}$ in the Hamiltonian $H_{Hubbard}$ of the solid-state material sample model may be expressed as:

$$\sum_{i,j,\sigma} t_{ij} S_i^z f_{i,\sigma}^+ S_j^z f_{j,\sigma}$$

In some embodiments, the term $S_i^z f_{i,\sigma}^+ S_j^z f_{j,\sigma}$ in the infinite sum $\sum_{i,j,\sigma} t_{ij} S_i^z f_{i,\sigma}^+ S_j^z f_{j,\sigma}$ may be approximated through a mean-field decoupling of the pseudo-fermion and spin degrees of freedom, for example as follows:

$$S_i^z f_{i,\sigma}^+ S_j^z f_{j,\sigma} \approx \langle S_i^z S_j^z \rangle f_{i,\sigma}^+ f_{j,\sigma} + S_i^z S_j^z \langle f_{i,\sigma}^+ f_{j,\sigma} \rangle - \langle S_i^z S_j^z \rangle \langle f_{i,\sigma}^+ f_{j,\sigma} \rangle$$

In some embodiments, this leads to mapping the Hamiltonian operator H onto a sum of two self-consistent Hamiltonians $H_f$ and $H_s$:

$$H \approx H_f + H_s$$

wherein

$$H_f = \sum_{i,j,\sigma} Q_{ij} f_{i,\sigma}^+ f_{j,\sigma}$$

is a free fermionic Hamiltonian with a renormalized hopping, with

$$Q_{ij} = t_{ij}\langle S_i^z S_j^z \rangle$$

and

$$H_s = \sum_{i,j} J_{ij} S_i^z S_j^z + \frac{U}{4} \sum_i S_i^x$$

is a Hamiltonian operator that describes a longitudinal spin interaction (through the term $\sum_{i,j} J_{ij} S_i^z S_j^z$) and a transverse field (through the term $\frac{U}{4}\sum_i S_i^x$) (e.g. a transverse field Ising Hamiltonian), with

$$J_{ij} = \sum_{\sigma} t_{ij}\langle f_{i,\sigma}^+ f_{j,\sigma} \rangle$$

Therefore, in some embodiments, depending on the model used, the Hamiltonian operator describing a longitudinal spin interaction and a transverse field may comprise a combination of a longitudinal spin interaction term and a transverse field term, such as for example in some embodiments a transverse field Ising Hamiltonian operator.

As a consequence, in one or more embodiments, mapping the Hubbard model onto a plurality of Hamiltonian operators within slave spin theory amounts to solving these two self-consistently defined problems: computing the Hamiltonian operator $H_f$ (sometimes referred to herein as the "pseudo-fermion problem," and computing the Hamiltonian operator $H_s$ (sometimes referred to herein as the "spin problem").

In one or more embodiments, solving the Hamiltonian $H$ approximated by the combination $H_f + H_s$ may be performed in an iterative fashion, for example as follows:

Starting from an initial estimate for the renormalized hopping $Q$ to define $H_f$ and thus initiate the self-consistent computation,

(i) compute the correlation function $\langle f_{i,\sigma}^+ f_{j,\sigma} \rangle$ of the pseudo-fermion problem, which is also needed to define the spin interaction $J_{ij}$ in the spin problem. In some embodiments, the computation of such correlation function $\langle f_{i,\sigma}^+ f_{j,\sigma} \rangle$ and the resolution of the pseudo-fermion problem, hence the computation of the free fermionic Hamiltonian operator with a renormalized hopping $H_f$, may be advantageously performed on a classical (non-quantum) computer, for example using a Bogoliubov transformation (which is described in further details in Appendix B.1). Therefore, in some embodiments, an estimate of a property of the free fermionic Hamiltonian operator with renormalized hopping of the plurality of Hamiltonian operators may be computed using a non-quantum processor. In some embodiments, the estimate of the quantum phase parameter which is output by the proposed scheme may further be based on the estimate of the property of the free fermionic Hamiltonian operator (which may be computed using a non-quantum processor);

(ii) solve the spin problem, that is, compute the Hamiltonian operator $H_s$ that describes a longitudinal spin interaction and a transverse field.

In one or more embodiments, the spin problem, which is computationally very complex for a computation on a classical (non-quantum) computer to be considered, may advantageously be approximated into a form that is well suited for solving the spin problem on a quantum computer.

An advantage of the proposed scheme is therefore that, for example as part of solving the spin problem, an estimate of a property of the Hamiltonian operator describing a longitudinal spin interaction and a transverse field stated in the spin problem may be computed on a quantum computer, whereas it may be considered too computationally complex to be computed using a classical (non-quantum) computer.

The present subject disclosure therefore advantageously allows computing an estimate of a property of a Hamiltonian operator describing a longitudinal spin interaction and a transverse field comprised in an estimate of a Hamiltonian operator $H$ of a solid-state material lattice model of a solid-state material sample, by putting such estimate in a form that is well suited for computation on a quantum computer. As a consequence, a parameter characterizing a property of a quantum phase of a solid-state material sample (sometimes referred to herein as a "quantum phase parameter") can advantageously be computed (using, at least for part of the computation, a quantum computer).

In one or more embodiments, an estimate of the hopping parameter t for the solid-state material sample may be obtained by measuring the estimate of hopping parameter $t$ on the solid-state material sample.

Likewise, in one or more embodiments, an estimate of the on-site interaction parameter $U$ for the solid-state material sample may be obtained by measuring the estimate of the on-site interaction parameter $U$ on the solid-state material sample.

As a consequence, depending on the embodiments, one or more of the estimates of the of hopping parameter $t$ and the on-site interaction parameter $U$ may be determined by performing experimental measurements on the solid-state material sample.

In one or more embodiments, the Hamiltonian operator of the Fermi-Hubbard model may be solved by performing one or more iterations of a model solving loop (which may itself comprise one or more model solving loops (which may be referred to herein as "inner loop" and "outer loop")), an iteration of the model solving loop comprising one or more of the following acts: computing, using the non-quantum processor, the correlation function $\langle f^+ f \rangle_{nn}$ of the transverse field Ising Hamiltonian operator based on an initial value of a renormalized hopping parameter $Q$, Using the correlation function $\langle f^+ f \rangle_{nn}$ of the transverse field Ising Hamiltonian operator to compute, using the quantum processor, a correlation function $\langle S^z S^z \rangle_{nn}$ of the free fermionic Hamiltonian operator, and Compute a value of the renormalized hopping parameter $Q$ based on the correlation function $\langle S^z S^z \rangle_{nn}$.

In one or more embodiments, a computation problem with an infinite number of spins may be reduced into a computation problem with a finite number of spins, as follows:

In one or more embodiments, since the Hamiltonian operator $H_s$ describing a longitudinal spin interaction and transverse field is of infinite size (as it includes infinite sums respectively describing the longitudinal spin interaction among an infinity of fermions and the transverse field generated by such infinity of fermions), it may advantageously be reduced to a finite-size problem by using a cluster mean-field approximation.

For example, in some embodiments, the Hamiltonian operator $H_s$ may be approximated by the following finite spin problem Hamiltonian operator $H_s^C$:

$$H_s \approx H_s^C = \sum_{i,j \in C} J_{ij} S_i^z S_j^z + \frac{U}{4} \sum_{i \in C} S_i^x + \sum_{i \in C} h_i S_i^z$$

where C denotes the set of $N$ cluster sites, and $h_i = 2z_i \overline{J_m}$, is the self-consistent mean field that mimics the influence of the infinite lattice, and
$z_i$ is the number of neighbors of site $i$ outside the cluster,

$$\bar{J} = \frac{1}{N_p} \sum_{\langle i,j \rangle \in C} J_{i,j}$$

corresponds to the average nearest-neighbor coupling ($N_p$ is the number of nearest-neighbor links inside the cluster), and

$$\bar{m} = \frac{1}{N} \sum_{i \in C} \langle S_i^z \rangle$$

is the average magnetization (for example the average over all sites of the site magnetization).

In one or more embodiments, this finite spin problem Hamiltonian operator model $H_s^C$ may be solved iteratively by starting from a guess for the mean field $\overline{m}$.

In one or more embodiments, for a given value of this mean field, the finite spin problem defined by $H_s^C$ may be solved using a quantum algorithm, as described below.

This yields the correlation function $\langle S_i^z S_j^z \rangle$ and closes the self-consistent loop, which runs until convergence.

At convergence, we can extract useful observables of the original Hubbard model.

For instance, the quasiparticle weight $Z$ of the original model, which measures the quantum coherence of the fermionic excitations, can be obtained based on the spin model's magnetization, for example through the following equation:

$$Z = \bar{m}^2$$

Therefore, the self-consistent computation, which allows obtaining the average magnetization, also allows obtaining in some embodiments the quasiparticle weight $Z$ (as an exemplary quantum phase parameter) through the above relation. For example, in some embodiments, obtaining site-resolved magnetizations $\langle S_i^z \rangle$ according to the proposed scheme may advantageously allow obtaining site-resolved quasiparticle weights.

In one or more embodiments, a solution of the (cluster) spin problem, $H_s^C$ that may correspond to a quantum algorithm.

In some embodiments, solving the transverse-field Ising model on a quantum computer may advantageously be considered based on the similarities between formulations of the transverse-field Ising model and a Hamiltonian realized by Rydberg atoms trapped with optical tweezers, such as for example:

$$\widehat{H}_{Rydberg} = \sum_{i \neq j} \frac{C_6}{\left| r_i - r_j \right|^6} \hat{n}_i \hat{n}_j + \frac{\hbar \Omega(\tau)}{2} \sum_i \hat{S}_i^x - \hbar \delta(\tau) \sum_i \hat{n}_i$$

where $\Omega(\tau)$ and $\delta(\tau)$ are the time-dependent Rabi and detuning drives, and $C_6$ the magnitude of the interatomic van der Waals interactions; $\hat{n}_i = (I_i + S_i^z)/2$.

It may be noted that a difference between $\hat{H}_{Rydberg}$ and $H_s^C$ is the sign of the interaction: it is positive for Rydberg atoms, negative (since usually $t_{ij} < 0$) for the slave spin problem.

In one or more embodiments, use of the Rydberg platform may nevertheless be considered for solving the (cluster) spin problem $H_s^C$: instead of computing the (cluster) spin problem $H_s^C$ in ground state, the (cluster) spin problem $H_s^C$ may be computed in its most excited state, which can be performed in some embodiments using an annealing procedure:

In some embodiments, the annealing procedure may start from drive parameters $\Omega(\tau = 0) = 0$ and a large negative $\delta(\tau = 0)$ so that the system's native initial state $|\psi_{start}\rangle = |g\rangle^{\otimes N}$ is the most excited state of the initial Hamiltonian.

Thereafter, the annealing procedure may comprise, over a long enough annealing time, linearly ramping the Rabi and detuning drives to reach final values such as for example the following final values:

$$\hbar \Omega(\tau_{max}) = \frac{U}{2}$$

$$\hbar \delta_i(\tau_{max}) = \sum_{j \neq i} \frac{C_6}{r_{i,j}^6} + 4\bar{J}\bar{m}z_i$$

In some embodiments, the atom positions may be optimized using the following approximation:

$$\frac{C_6}{r_{i,j}^6} \approx -4J_{i,j}$$

(the optimization of which is described in further details in Appendix C.1). As a consequence, the final (cluster) spin problem $H_s^C$ Hamiltonian may be approximated as follows: $H_s^C - H_{spin}^C$ .

Hence, following the adiabatic theorem (applied to the most excited state), the procedure should (approximately) bring the system to the most excited state of $-H_{spin}^C$ and hence the ground state of $H_{spin}^C$ . We can finally measure the spin-spin correlation function on this state, that is, the quantity $\langle S_i^z S_j^z \rangle$ , which may be used in some embodiments to update the quantity $Q$ that may be used to define $H_f$.

In one or more embodiments, the estimated quantum phase parameter obtained using a method according to embodiments of the present subject disclosure may characterize the behaviour of the solid-state material sample in an equilibrium state of the solid-state material sample.

In such embodiments, an estimate of a property of the Hamiltonian operator describing a longitudinal spin interaction and a transverse field, such as for example the finite spin problem Hamiltonian operator $H_s^C$ , may be computed using a quantum processor, for example using arrays of Rydberg atoms.

In some embodiments, one or more instances of the computation may be performed with respective values of the local Hubbard interaction parameter $U$ to obtain an estimate of a quantum phase parameter of the solid-state material such as the quasiparticle weight $Z$.

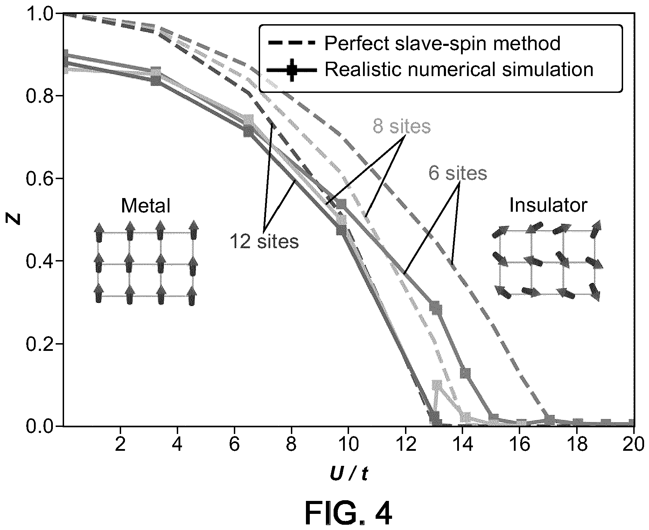

In some embodiments, a plurality of instances of the computation for respective values of the local Hubbard interaction parameter may be performed to obtain the evolution of the quasiparticle weight $Z$ as a function of $U$, as illustrated in Figure 4, for exemplary cluster sizes, and hence exemplary number of atoms, of 4, 6, 8 and 12.

Figure 4 shows curves of estimated values of quasiparticle $Z$ as a function of the ratio $U/t$, for different numbers of sites in the lattice.

In some embodiments, the quantum computer used for implementing the proposed method may be viewed as a quantum system operating on a number of qubits corresponding to the number of sites in the considered lattice model: 4 sites - 4 qubits, 6 sites - 6 qubits, 8 sites - 8 qubits, and 12 sites - 12 qubits.

Fig. 4 shows experimental results obtained with an exemplary device characterized by the following parameters: maximal annealing time $t_{max} = 4\mu s$, laser dephasing strength $\gamma = 0.02$ $MHz$, number of shots $N_s = 150$, and false-positive and false-negative rates $\varepsilon = \varepsilon' = 3\%$.

The major experimental limitations were considered in order to account for the true potential of current devices: dephasing noise, shot noise, measurement error, global detuning, finite annealing times $\tau_{max}$ and imperfect positioning of the atoms to reproduce the right magnetic coupling (which is described in further details in Appendix C). Dephasing noise stems from an imperfect control of the laser, shot noise comes from the fact that quantum mechanical observables need to be estimated by sampling from a finite number of measurements, leading to statistical uncertainty. Finite annealing times may lead to a final state that deviates from the perfect ground state due to a violation of the aforementioned adiabatic theorem.

As shown on Fig. 4, despite these limitations resulting in a few points being remote from the noiseless result due to error repetitions within all loops, the obtained quasiparticle weight estimate (solid lines on Fig. 4) is close to the one obtained with a perfect solution of the spin model (dashed lines on Fig. 4).

Therefore, in some embodiments, a Rydberg platform can advantageously be used to get an estimate of the so-called Mott transition for the solid-state material sample, that is, of the value $U_C$ when $Z$ vanishes and the system turns Mott insulating. The Mott transition value may be seen as a critical value of the $U$ parameter for which the quasiparticle weight $Z$ becomes small (can be considered as approximately zero), which corresponds to a phase transition between the solid-state material being Mott conductor and the solid-state material being Mott insulating.

While for the exemplary half-filled, single-band model described above, conventional methods have been proposed to solve the spin model, other regimes, such as doped regimes, multiorbital models, and dynamical regimes, are less readily amenable to a controlled classical computation and may therefore fully benefit from methods according to embodiments of the present subject disclosure.

In the following, experimental results are provided for dynamical regimes of a solid-state material sample.

In one or more embodiments, the estimated quantum phase parameter obtained using a method according to embodiments of the present subject disclosure may characterize the behaviour of the solid-state material sample in an out of equilibrium state of the solid-state material sample.

The use of quantum processors as proposed in the present subject disclosure is particularly advantageous for addressing the slave-spin problem computation for a system subjected to a dynamical setting to be put in an out of equilibrium state.

For example, starting from a noninteracting ground state ($U = 0$), the value of the local interaction may suddenly be switched on to a final value $U_f$.

Typically, based on previous experiments, one would expect to observe collapse and revival oscillations of various observables in the $U_f \gg U_c$ regime, with a $2\pi/U_f$ period, and a damping that increases with bandwidth.

In the $U_f \ll U_c$ regime, overdamped oscillations have been observed.

The following discusses this phenomenology in the time evolution of the quasiparticle weight $Z$:

In one or more embodiments, interaction quenches may be implemented within slave-spin applied to the single-site Hubbard model at half-filling. Translation invariance on the lattice makes the dynamics of pseudo-fermions trivial when starting from an eigenstate, as described in Annex A4 of the present subject disclosure.

Thus, only the dynamics of the spin model are of interest: the procedure boils down to quenching the value of the transverse field in the equation $H_s^C = \sum_{i,j} J_{ij} S_i^z S_j^z + \frac{U}{4} \sum_{i \in C} S_i^x + \sum_{i \in C} h_i S_i^z$ from 0 to $U_f/4$. On a target Rydberg platform, the Rabi frequency may be switched from zero up to the desired value to obtain $U_f$. In practice, the switch-on time is not instantaneous but very fast (about 50 $ns$ to switch from 0 $MHz$ to $U_f = 2MHz$).

One can directly measure (Sf) on the device for different evolution times. The main limiting factor is thus the measurement rate, in addition to the aforementioned sources of noise.

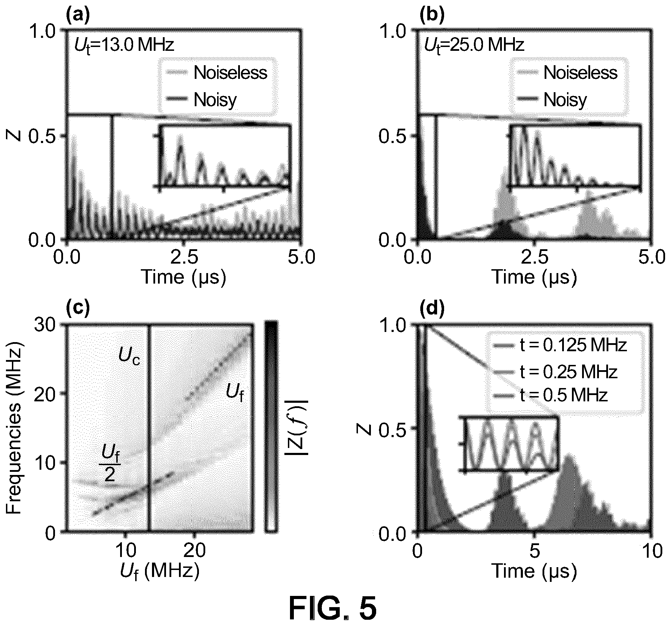

Figures 5(a) - 5(d) show an exemplary dynamical response of a quasiparticle weight parameter $Z$ after an interaction quench in accordance with one or more embodiments of the present subject disclosure for an exemplary $N = 12$ spin cluster.

Figures 5(a) and 5(b) show oscillations observed numerically for a cluster of 12 sites, with and without noise. The upper panels present the oscillation of $Z$ as function of time after a quench to $U_f = 13$ $MHz$ (Fig. 5(a)) and to $U_f = 25$ $MHz$ (Fig. 5(b)).

Figs. 5(a) and 5(b) respectively show an exemplary time evolution of Z for $U_f = 13$ $MHz$ (Fig. 5(a)) and for $U_f = 25$ $MHz$ (Fig. 5(b)). On these figures, the light line shows the noiseless annealing solution, while the dark line shows results of a numerical simulation on Rydberg atoms device ($\gamma = 0.02$ $MHz$, $\varepsilon = \varepsilon' = 3\%$, $N_s = 150$ shots, realistic Ising interactions and a global detuning are imposed).

As shown on Fig. 5(a), the phase transition for such a cluster is $U_c \approx 13.5$. Fig. 5b shows damped oscillation that can be observed in the case of $U_f = 25$ $MHz$ ($U_f \gg U_c$), whether in the noiseless or the noisy setting.

Because of dephasing noise of the experiment, the agreement between the noiseless and noisy curve becomes worse with time, as the effects of noise accumulate with time.

However, during the first $\mu s$ of observations, we recover the perfect signal and the estimation of the oscillation frequency is possible (insets in (a) and (b)). For $U_f = 13$ $MHz$ ($U_f \approx U_c$), we see that $Z$ quickly reaches a value $\ll 0.1$ (slightly higher than the $Z$ obtained for this value of $U$ at equilibrium), around which it oscillates.

Fig. 5(c) shows the Fourier transform amplitude $|Z(f)|$ for several $U_f$. The vertical black line shows the equilibrium critical value as computed using the proposed method illustrated by Fig. 4, and described in relation thereto.

Figure 5 (c) exhibits the Fourier transform of $Z(\tau)$ for various $U_f$ for the exact slave-spin method (namely with an exact solution of the spin model). For $U_f < U_c$, components at $\omega = U_f/2$ can be identified along with other contributions, while for $U_f > U_c$, $\omega = U_f$ end up dominating the spectrum. This is expected from the physics of the Mott transition in the Hubbard model: above the transition, the single-particle spectrum displays a Mott gap of $U_f$ while below it excitations between the quasiparticle band and the emerging Hubbard bands (with energy $U_f/2$), and within the quasiparticle band, are possible.

Figure 5 (d) shows an exemplary impact of the hopping terms t on the damping of the response of $Z$ after a quench ($U_f = 13 \approx U_c$). The three lines with respective gray shades show the result for $t = 0.125$, $0.25$, and $0.5$ $MHz$, respectively.

Fig. 5(d) confirms the expected increase of the damping of oscillations with the hopping strength $t$.

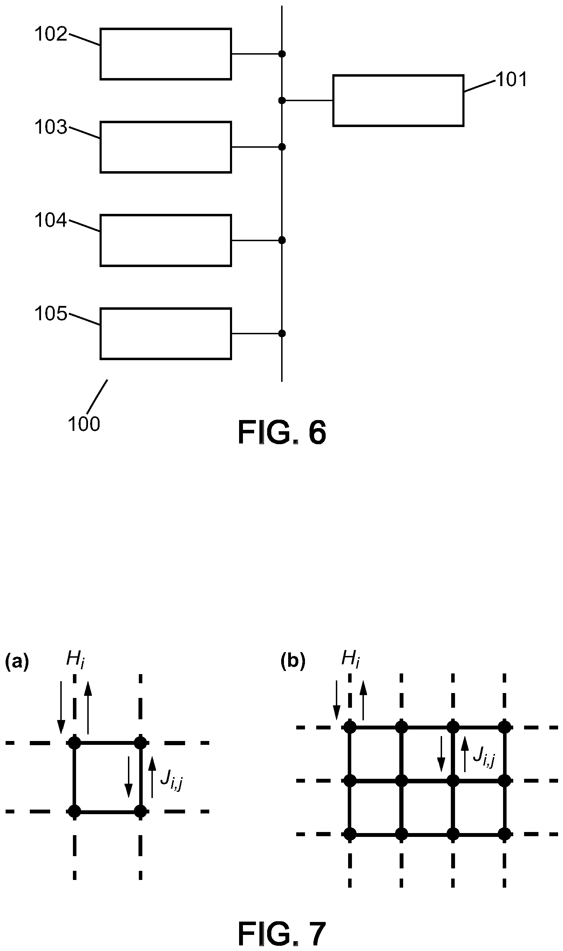

Fig. 6 illustrates an exemplary apparatus 100 configured to implement a method of determining a parameter characterizing a property of a quantum phase of a solid-state material sample in accordance with embodiments of the present subject disclosure. The apparatus 100 may, depending on the embodiment, be comprised in a quantum computing apparatus, a HPC cluster, an electronic circuit, an electronic board, an electronic component, a chip, a QPU or any other suitable processing platform.

The apparatus 100, which may comprise one or more computers, includes a control engine 101, a Hamiltonian estimation engine 102, a computing engine 103, a data interface engine 104, and a memory 105.

In the architecture illustrated on Fig. 6, all of the Hamiltonian estimation engine 102, computing engine 103, data interface engine 104, and memory 105 are operatively coupled with one another through the control engine 101.

In one or more embodiments, the Hamiltonian estimation engine 102 may be configured to perform various functions or embodiments provided in the present subject disclosure, including with respect to one or more of obtaining (or determining) an estimate of a Hamiltonian operator $H$ of a solid-state material lattice model of the solid-state material sample, and mapping the estimate of the Hamiltonian operator H onto a combination of a plurality of Hamiltonian operators comprising a Hamiltonian operator describing a longitudinal spin interaction and a transverse field. In some embodiments, the Hamiltonian estimation engine 102 may be implemented in software and incorporated in a computing machine running on a hybrid computer, configured according to embodiments of the present subject disclosure.

In one or more embodiments, the computing engine 103 may be configured with one or more quantum processing resources (e.g. one or more QPUs) to perform various functions or embodiments provided in the present subject disclosure, including with respect to computing, using a quantum processor, an estimate of a property of the Hamiltonian operator describing a longitudinal spin interaction and a transverse field. Further, in one or more embodiments, the computing engine 103 may be further configured with one or more classical processing resources (e.g. one or more CPUs) to perform various functions or embodiments provided in the present subject disclosure, including with respect to computing an estimate of the quantum phase parameter as a property of the Hamiltonian operator $H$, based on the estimate of the property of the Hamiltonian operator describing the longitudinal spin interaction and the transverse field. In addition, in one or more embodiments, the computing engine 103 may be further configured with one or more classical processing resources (e.g. one or more CPUs) to perform various functions or embodiments provided in the present subject disclosure, including with respect to computing an estimate of a property of the free fermionic Hamiltonian operator with renormalized hopping comprised in the plurality of Hamiltonian operators.

In one or more embodiments, the data interface engine 104 may be configured to provide a data interface to the apparatus 100, including for exchanging data with another apparatus or receiving, through a user input interface, input data to be used for implementing a method according to embodiments of the present subject disclosure.

The control engine 101 includes a processor, which may be any suitable microprocessor, microcontroller, Field Programmable Gate Arrays (FPGA), Application Specific Integrated Circuits (ASIC), Digital Signal Processing chip, and/or state machine, or a combination thereof. According to various embodiments, one or more of the computers can be configured as a multi-processor computer having multiple processors for providing parallel computing. The control engine 101 may also comprise, or may be in communication with, computer storage media, such as, without limitation, the memory 105, capable of storing computer program instructions or software code that, when executed by the processor, causes the processor to perform the elements described herein. In addition, the memory 105 may be any type of data storage computer storage medium, capable of storing multimedia content data, service data, service list data, customization data, personalization data and/or channel data for use according to one or more embodiments of the present subject disclosure, coupled to the control engine 101 and operable with the data interface engine 104, the computing engine 103, and the Hamiltonian estimation engine 102 to facilitate processing of data stored in association therewith.

In embodiments of the present subject disclosure, the apparatus 100 is configured for performing the processing methods described herein.

It will be appreciated that the apparatus 100 shown and described with reference to Fig. 6 is provided by way of example only. Numerous other architectures, operating environments, and configurations are possible. Other embodiments of the apparatus may include fewer or greater number of components and may incorporate some or all of the functionality described with respect to the apparatus components shown in Fig. 6. Accordingly, although the control engine 101, data interface engine 104, computing engine 103, Hamiltonian estimation engine 102, and memory 105 are illustrated as part of the apparatus 100, no restrictions are placed on the location and control of components 101 - 105. In particular, in other embodiments, components 101 - 105 may be part of different entities or computing systems.

Embodiments of the present subject disclosure advantageously provide a hybrid quantum-classical method to study the equilibrium and dynamics of a prototypical model for strongly correlated fermions, the Hubbard model. Thanks to the use of an advanced mapping, such as for example in some embodiments the slave-spin method, that turns the difficult fermionic problem at hand into a free, and thus efficiently tractable, fermion problem that is self-consistently coupled to an interacting, yet local spin problem, the proposed method does not suffer from the usual overheads of translating fermionic problems to spin problems, namely long quantum evolutions (due to nonlocal spin terms) or auxiliary quantum degrees of freedom, even though it uses in some embodiments a spin-based quantum processor. This locality makes this spin problem well suited for current quantum processors based on spins (aka qubits).

In one or more embodiments, an analogue quantum processor (as opposed to a gate-based quantum processor), such as for example made of Rydberg atoms, may advantageously be used to solve the spin problem. Despite being a priori restricted in the class of the problems that it can deal with (because of the limited number of knobs in the Hamiltonian), the Rydberg platform is particularly well suited for the spin problem at hand, and therefore for implementing embodiments of the present subject disclosure, because its Hamiltonian can be made to (almost exactly) coincide with the effective spin Hamiltonian to be solved.

Moreover, its analogue character allows one to circumvent the usual issues associated with gate-based algorithms, like trotterization when performing time evolution, or the variational aspects inherent to many NISQ algorithms like VQE or its temporal counterparts. Further, the number of Rydberg atoms that can be controlled in current experiments advantageously allows to tackle problem sizes that are very hard to reach using classical methods.

Although this subject disclosure has been disclosed in the context of certain preferred embodiments, it should be understood that certain advantages, features and aspects of the systems, devices, and methods may be realized in a variety of other embodiments. Additionally, it is contemplated that various aspects and features described herein can be practiced separately, combined together, or substituted for one another, and that a variety of combination and sub-combinations of the features and aspects can be made and still fall within the scope of the subject disclosure. Furthermore, the systems and devices described above need not include all of the modules and functions described in the preferred embodiments.

Information and signals described herein can be represented using any of a variety of different technologies and techniques. For example, data, instructions, commands, information, signals, bits, symbols, and chips can be represented by voltages, currents, electromagnetic waves, magnetic fields or particles, optical fields or particles, or any combination thereof.

Depending on the embodiment, certain acts, events, or functions of any of the methods described herein can be performed in a different sequence, may be added, merged, or left out all together (e.g., not all described acts or events are necessary for the practice of the method). Moreover, in certain embodiments, acts or events may be performed concurrently rather than sequentially.

Appendix A: Summary of the slave-spin method

*A.1 - Main equations*

Any suitable form of slave spins, such as the form of slave spins according to the $Z_2$ slave spin theory (as described in the following), may be used depending on embodiments of the present subject disclosure.

In the following, embodiments of the present subject disclosure are described in the exemplary case where the $Z_2$ slave spin theory is used. However, a person of ordinary skill in the art would understand that the processes, apparatuses and computer programs of the present subject disclosure may be implemented based on any suitable slave spin scheme, such as for example the slave spin scheme described in Annex A.3, and that such proposed processes, apparatuses and computer programs of the present subject disclosure are not limited to the use of the $Z_2$ slave spin theory, which is provided as an example only.

The main operations (acts) of the slave-spin method in some embodiments may be as follows:

In some embodiments, the fermionic operator $d^\dagger$ may be replaced by the tensor product of a pseudo fermion operator (that follows the same anticommutation rules as $d^\dagger$) and an auxiliary spin field, for example as follows:

$$d^\dagger_{i\sigma} = S^z_i f^\dagger_{i\sigma} \quad (A1)$$

where Sf is the Pauli-Z operator at site i (later $S^a_i$, with $a \in \{x, y, z\}$, will denote the Pauli spin operators), and $f^\dagger_{i\sigma}$ and $f_{i\sigma}$ denote fermionic operators called pseudo-fermions (the symbol "†" designating the complex conjugate transpose of a matrix). The d and f operators obey fermionic anticommutation relations due to the spin commutation relations.

As a result of substituting, in the Hamiltonian operator $H_{Hubbard}$, the original fermionic operators by new spin and pseudo fermion degrees of freedom, the Hilbert space where the new Hamiltonian, $H'_{Hubbard}$, acts may be enlarged. In some embodiments, the original problem $H_{Hubbard}$ may be mapped to a Hilbert space of the same size by looking at a restriction of the new Hamiltonian, $H'_{Hubbard}$, on a restricted portion of the new Hilbert state, which may be referred to herein as the "physical subspace". In some embodiments, this mapping may be achieved by imposing a constraint, for example as follows: on each site i, imposing the relation:

$$\left(n^f_{i\uparrow} + n^f_{i\downarrow} - 1\right)^2 = \frac{S^x_i + 1}{2} \quad (A2)$$

to hold for the "physical states".

Among the eight possible local states, only four states (i.e. the same number of original local states) verify this exemplary constraint:

$$|n_i^d = 0\rangle = |S^x = 1, n_i^f = 0\rangle \qquad \text{(A3a)}$$

$$|n_{i,\sigma}^d = 1\rangle = |S^x = -1, n_{i,\sigma}^f = 1\rangle, \sigma = \uparrow, \downarrow \qquad \text{(A3b)}$$

$$|n_i^d = 2\rangle = |S^x = 1, n_i^f = 2\rangle \qquad \text{(A3c)}$$

Assuming this constraint is satisfied in the physical subspace, the original Hubbard Hamiltonian, expressed as

$$H_{\text{Hubbard}} = \sum_{i,j,\sigma} t_{i,j} d_{i\sigma}^\dagger d_{j\sigma} + \frac{U}{2} \sum_i (n_i^d - 1)^2 + (\mu - \frac{U}{2}) \sum_i n_i^d \qquad \text{(A4)}$$

may be transformed in some embodiments to the following transformed Hamiltonian:

$$H'_{\text{Hubbard}} = \sum_{i,j,\sigma} t_{i,j} S_i^z S_j^z f_{i,\sigma}^\dagger f_{j,\sigma} + \frac{U}{2} \sum_i \left(\frac{S_i^x + 1}{2}\right) + (\mu - \frac{U}{2}) \sum_i (n_{i,\uparrow}^f + n_{i,\downarrow}^f) \qquad \text{(A5)}$$

via substitution of equality [Equation A2] in the interaction term of the Equation A4. It is straightforward to see that $n_i^d = n_i^f$ considering Equations A3 above. At this point, no approximation has been made.

In some embodiments, the next operation (act) may comprise: decoupling fermions and spins with a mean-field approach:

$$S_i^z S_j^z f_{i,\sigma}^\dagger f_{j,\sigma} \approx \langle S_i^z S_j^z \rangle f_{i,\sigma}^\dagger f_{j,\sigma} + S_i^z S_j^z \langle f_{i,\sigma}^\dagger f_{j,\sigma} \rangle - \langle S_i^z S_j^z \rangle \langle f_{i,\sigma}^\dagger f_{j,\sigma} \rangle \qquad \text{(A6)}$$

Therefore, Equation A5 can be expressed as a combination (in this exemplary case a sum) of two Hamiltonian operators (neglecting constant terms and considering half-filling):

$$H'_{\text{Hubbard}} = H_s + H_f$$

with

$$H_s = \sum_{i,j} t_{i,j} \langle f_{i,\sigma}^\dagger f_{j,\sigma} \rangle S_i^z S_j^z + \frac{U}{4} \sum_i S_i^x$$

a Transverse Field Ising Model (TFIM) and

$$H_f = \sum_{i,j,\sigma} t_{i,j} \langle S_i^z S_j^z \rangle f_{i,\sigma}^\dagger f_{i,\sigma}$$

describing the free renormalized electrons.

In some embodiments, the correlators $\langle f_{i,\sigma}^\dagger f_{j,\sigma} \rangle$ and $\langle S_i^z S_j^z \rangle$ may be obtained via auto-coherent loops until convergence is reached.

*A.2 - Fulfilment of the constraint*

In some embodiments, when performing the loops described above, one may ensure that the constraint of Equation A2 is imposed on each site.

In practice, the mean-field simplification may for example lead to the following equation:

$$\langle (n_{i,\uparrow}^f + n_{i,\downarrow} - 1)^2 \rangle_f = \langle \frac{S_i^x + 1}{2} \rangle_s \quad (A7)$$

In one or more embodiments, this equality may be enforced on all sites, for example by using a Lagrange multiplier $\lambda_i$: For example, one may add a term $H_\lambda = \sum_i \lambda_i ((n_i - \quad 1)^2 - \frac{S_i^x + 1}{2})$ to $H_s + H_f$, and optimize the corresponding cost function.

In particle-hole symmetric cases (which includes our setting, namely the single-orbital, half-filled Hubbard model on a square - i.e bipartite - lattice), $\lambda_i$ may be set to zero to respect the symmetry of the energy spectrum around 0.

*A.3 - Variants. Towards a multiorbital case*

In the following, embodiments of the proposed method that use a slave spin other than the $Z_2$ slave spin theory are described.

In one or more embodiments, another related slave-spin approach may be used, which consists in enlarging the Hilbert space with the spin operator $S_{i,\sigma}^z$ such as

$$d_{i,\sigma}^\dagger = f_{i,\sigma}^\dagger S_{i,\sigma}^z \quad (A8)$$

In these embodiments, the physical states may be described as follows:

$$|n_{i,\sigma}^d = 1\rangle \Leftrightarrow |n_{i,\sigma}^f = 1, S_{i,\sigma}^z = 1\rangle$$

And

$$|n_{i,\sigma}^d = 0\rangle \Leftrightarrow |n_{i,\sigma}^f = 0, S_{i,\sigma}^z = -1\rangle$$

The constraint to be satisfied in order to only span physical states may then be expressed as:

$$n_{i,\sigma} = S_{i,\sigma}^z + \frac{1}{2}$$

While this method lends itself quite naturally to multiorbital models, the additional $\sigma$ dependency of the slave-spin operators (as compared to the $Z_2$ slave spins described in relation to embodiments of the present subject disclosure) may also lead to an effective model which may in some embodiments advantageously be mapped to existing quantum computation platforms as proposed in the present subject disclosure.

*A.4 - Dynamics in slave-spin theory*

In the following, a description of the slave-spin formalism extending to the time-dependent case is provided.

After the introduction of the slave variables, we are considering the Hamiltonian:

$$H'_{Hubbard} = \sum_{i,j\sigma} S_i^z S_j^z f_{i\sigma}^\dagger f_{j\sigma} + \frac{U}{4} \sum_i S_i^x \quad (A9)$$

(Equation A5 at half-filling and neglecting the constants).

At the mean-field level, the time-dependent solution of the Schrödinger equation may be of the following form:

$$|\Psi(\tau)\rangle = |\Phi_f(\tau)\rangle |\Psi(\tau)\rangle$$

With $|\Phi_f(\tau)\rangle$ (the time will be defined as $\tau$ to avoid confusion with the hopping) governed by a Schrödinger evolution with time-dependent Hamiltonians:

$$H_f(t) \quad = \sum_{i,j,\sigma} t_{i,j} \langle S_i^z S_j^z \rangle(\tau) f_{i\sigma}^\dagger f_{j\sigma}$$

$$H_s(\tau) \quad = \sum_{i,j\sigma} t_{ij} S_i^z S_j^z \langle f_{i\sigma}^\dagger f_{j\sigma} \rangle(\tau) + \frac{U}{4} \sum_i S_i^x \qquad (A10)$$

The initial state may be of the following form:

$$|\Psi(\tau = 0)\rangle = |\Phi_f(\tau = 0)\rangle |\Psi(\tau = 0)\rangle$$

with $|\Psi(\tau = 0)\rangle$ (respectively $|\Phi_f(\tau = 0)\rangle$) corresponding to the ground states of $H_f(\tau < 0)$ (respectively $H_s(\tau < 0)$) found with the mean-field slave-spin procedure.

To solve these coupled equations, we a priori need to compute correlators $\langle S_i^z S_j^z \rangle(\tau)$ and $\langle f_i^\dagger f_j \rangle(\tau)$ and use them to construct $H_f(\tau)$ and $H_s(\tau)$.

In some embodiments, the wavefunctions may have to be evolved in time in order to obtain correlators for a time $\tau$ + $d\tau$, and so on.

In some embodiments, the dynamics of the pseudo-fermions may be simpler in cases where the system is translation invariant (i.e $t_{ij} = t_{i-j}$).

In these cases, indeed,

$$\langle S_i^z S_j^z \rangle(\tau) = g_{i-j}(\tau)$$

Thus,

$$H_f(\tau) = \sum_{ij,\sigma} t_{i-j}\, g_{i-j}(\tau) f_{i\sigma}^\dagger f_{j\sigma} \qquad (A11)$$

This Hamiltonian operator is then diagonal in the Fourier space:

$$H_f(\tau) = \sum_{k,\sigma} \epsilon_k(\tau)\, f_{k\sigma}^\dagger f_{k\sigma} \qquad (A12)$$

wherein $\epsilon_k(\tau)$ designates the Fourier transform of $t_{i-j}g_{i-j}(\tau)$, and $n_k = f_{k\sigma}^\dagger f_{k\sigma}$, with $f_{k\sigma} \propto \Sigma_i e^{ikR_i} f_{i\sigma}$.

The Fock states of the system associated with the transformed operators, $f_k$, may be denoted herein as $|\Phi_\alpha\rangle$. They are the eigenstates of $H_f(\tau)$ at any time $\tau$.

The initial state $|\Phi_{\alpha 0}\rangle$ is the ground state of the system.

In some embodiments, by considering the time evolution of an arbitrary state $|\Phi_f(\tau)\rangle$, the following decomposition can be defined:

$$|\Phi_f(\tau)\rangle = \sum_\alpha c_\alpha(\tau) |\Phi_\alpha\rangle$$

Therefore,

$$\sum_\alpha i\, \partial_t c_\alpha(\tau) |\Phi_\alpha\rangle = \sum_\alpha c_\alpha(\tau) H_f(\tau) |\Phi_\alpha\rangle = \sum_\alpha c_\alpha(\tau) E_\alpha(\tau) |\Phi_\alpha\rangle \qquad (A13)$$

By a projection onto $\langle \Phi_\alpha(\tau)|$:

$$i\,\partial_t c_\alpha(\tau) = c_\alpha(\tau) E_\alpha(\tau) \qquad (A14)$$

Thus,

$$c_\alpha(\tau) = c_\alpha(\tau = 0) e^{-i\int_0^t E_\alpha(\tau')d\tau'}$$

Therefore, starting from the ground state, for $\alpha \neq \alpha_0$,

$$c_\alpha(\tau) = 0$$

And

$$c_{\alpha_0}(\tau) = e^{-i\phi(\tau)}$$

Therefore,

$$|\Phi_f(\tau)\rangle = e^{-i\phi(\tau)}|\Phi_f(\tau = 0)\rangle \qquad (A15)$$

and the renormalized fermionic system remains in the ground state up to global phase, meaning that $\langle f_i^\dagger f_j \rangle(\tau) = \langle f_i^\dagger f_j \rangle_0$ is independent of time. As a consequence, only the correlator $\langle S_i^z S_j^z \rangle(\tau)$ may be considered during the quench.

*A.5 - Dynamics and constraint fulfilment*

In embodiments using the $Z_2$ slave-spin theory, one may define the projector:

$$Q_i = (\frac{S_i^x + 1}{2} - (n_i - 1)^2)^2 = \frac{1 + S_i^x e^{i\pi n_i}}{2}$$

such that $Q_i|\Psi\rangle = 0$ respects the constraint set forth in Equation A3.

Using the fact that $[H_{Hubbard}', \Pi_i Q_i] = 0$, the constraint is fulfilled during the quench dynamics because $\Pi_i Q_i|\Psi(\tau = 0)\rangle = 0$.

Appendix B: Solution of the two coupled subproblems

Provided in this Appendix B is a description of a method that may be used in some embodiments to solve numerically the above-described equation $H_f = \sum_{i,j,\sigma} Q_{ij} f_{i,\sigma}^+ f_{j,\sigma}$ and equation $H_s = \sum_{i,j} J_{ij} S_i^z S_j^z + \frac{U}{4}\sum_i S_i^x$ in order to obtain matrices J and Q.

With respect to the Hamiltonian operator $H_s$, a description of the embedding of the cluster mean-field theory is also provided in the following.

*B.1 Exemplary method for solving the fermionic Hamiltonian $H_f$ for J: using the Bogoliuboy transformation*

In some embodiments, in order to compute $J_{ij}$, one may consider computing the following exemplary one-particle density matrix:

$$G_{ij}^\sigma =_f \langle \Psi_0 | f_{i,\sigma}^\dagger f_{i,\sigma} | \Psi_0 \rangle_f \qquad (B1)$$

$H_f$ may be rewritten as a matrix product:

$$H_f = F^\dagger Q F \qquad (B2)$$

with $F^\dagger = (f_{1,\downarrow}^\dagger, f_{1,\uparrow}^\dagger, f_{2,\downarrow}^\dagger, \ldots)$ and Q denoting a Hermitian, $N \times N$, matrix.

In some embodiments, the matrix Q may be diagonalized numerically: $Q = LDL^\dagger$, with $D = \mathrm{diag}\{\lambda_1, \lambda_2, \ldots, \lambda_N\}$.

Assuming an even number of sites, it may be considered that the trace of D vanishes, with as many negative diagonal coefficients ($\lambda_j < 0$) as there are positive diagonal coefficients ($\lambda_j > 0$).

In some embodiment, this may lead to define $C^\dagger = F^\dagger L \Leftrightarrow C = L^\dagger F$, and a diagonal form of $H_f$ may advantageously be obtained, for example as follows:

$$H_f = \sum_{i,\sigma} \lambda_i\, c_{i,\sigma}^\dagger c_{i,\sigma} \qquad (B3)$$

The ground-state energy of this Hamiltonian operator may be estimated in some embodiments based on the sum of negative diagonal coefficients $\lambda_i$. Therefore, the ground state may then be estimated by a Slater determinant $10101 \ldots 01\rangle_c$ in the c basis with 1 corresponding to negative energies, and 0 otherwise.

In some embodiments, in order to go back in the f basis, one may use the L matrices as follows:

$$G_{ij}^\sigma = \langle \psi_0 | \sum_{k,k',\sigma} L_{k,i}^\dagger L_{j,k'} c_{k,\sigma}^\dagger c_{k',\sigma} | \psi_0 \rangle \qquad (B4)$$

$$= \sum_{k,k',\sigma} \delta_{k,k'} L_{k,i}^\dagger L_{j,k'} \langle c_{k,\sigma}^\dagger c_{k',\sigma} \rangle \qquad (B5)$$

$$= \sum_{k,\sigma} L_{i,k}^* n_{k,\sigma} L_{k,j}^t \qquad (B6)$$

with $n_{k,\sigma}$ being equal to 1 for k indices corresponding to negative diagonal coefficients $\lambda_k < 0$.

In one or more embodiments, it may be considered that numerically, only matrices L and eigenvalues $\lambda_i$ may be required in order to compute J.

In some embodiments, this computational part may be implemented on a classical quantum computer as it has a polynomial complexity.

Going into the thermodynamic limit makes it easier as the system is really translation invariant and the Hamiltonian operator is then diagonal in the Fourier space.

In one or more embodiments, it may be opted to solve $H_f$ considering boundaries in order to show the effect of a finite-size system on embodiments of the proposed method.

*B.2 - Solving the spin Hamiltonian via a cluster mean-field approach*

In the following, a description of a method for computing $Q_{ij}$ according to embodiments of the present subject disclosure, is provided. In some embodiments, the proposed method uses the computations of the spin-spin correlation function $\langle S_i^z S_j^z \rangle$.

We consider a cluster of $N_x$ columns and $N_y$ rows (such as illustrated on Fig. 7 for an example) surrounded by a mean field.

Fig. 7 shows an exemplary schematic representation of a cluster geometry for (a) N = 4 sites and (b) 12 sites. The dashed black lines represent the interaction with the surrounding mean-field whereas the full black line show the interactions within the cluster. In the exemplary twelve sites lattice shown on Fig. 7(b), the two sites inside the cluster do not interact with the mean-field.

In some embodiments, the number of sites in the cluster can be estimated as: $N = N_x \times N_y$.

In some embodiments, the cluster mean-field approximation may lead to

$$S_i^z S_j^z \approx \langle S_i^z \rangle S_j^z + \langle S_j^z \rangle S_i^z - \langle S_i^z \rangle \langle S_j^z \rangle \qquad (B7)$$

where i(j) is inside the cluster at the border of it and j(i) is not.

In some embodiments, the mean-field parameter (Sf) may be approximated to be the same for a plurality of (all) sites in the thermodynamic limit.

As we consider in some embodiments finite-size systems, the following mean field value $\overline{m}$ may advantageously

be numerically computed:

$$\overline{m} = \frac{1}{N}\sum_{i=1}^{N}\langle S_i^z \rangle \qquad (B8)$$

Therefore, in some embodiments, the sum $\sum_{i,j} J_{ij} S_i^z S_j^z$ may expressed based on the following equation:

$$\sum_{i,j} J_{ij} S_i^z S_j^z = \sum_{i,j} J_{i,j}(m S_j^z + m S_i^z) = m \sum_{i,j} J_{i,j}(S_j^z + S_i^z)$$

neglecting constant terms.

However, the matrix element $J_{i,j}$ may be unknown for a site i inside the cluster and a site j outside of it. In the thermodynamic limit, all $J_{i,j}$ may be considered equal as it is the one-particle density matrix of a free fermionic system. Therefore, in some embodiments, a mean value of all $J_{i,j}$ for nearest neighbors inside the cluster may be used to estimate the interaction between sites inside and outside the cluster.

Let's then define:

$$\bar{J} = \frac{1}{N_p}\sum_{(i,j)} J_{i,j} \qquad (B9)$$

where the sum goes all over nearest neighbors in the cluster, and $N_p$ is the number of such pairs.

In the square lattice, it may be considered that each site has 4 nearest neighbors.

In some embodiments, a number $z_i$ may be defined as the number of neighbors outside the cluster for site i. For example, this number is equal to 0 for a site which has 4 neighbors inside the cluster.

In one or more embodiments, the mean-field term may be estimated as:

$$\sum_{i\in\mathcal{C}} h_i S_i^z = 2\bar{J}\overline{m}\sum_{i\in\mathcal{C}} z_i S_i^z \qquad (B10)$$

which leads to the approximation of the Hamiltonian operator $H_s$ in some embodiments by the following finite spin problem Hamiltonian operator $H_s^\mathcal{C}$:

$$H_s \approx H_s^\mathcal{C} = \sum_{i,j\in\mathcal{C}} J_{ij} S_i^z S_j^z + \frac{U}{4}\sum_{i\in\mathcal{C}} S_i^x + \sum_{i\in\mathcal{C}} h_i S_i^z$$

as discussed above.

## B.3 - Convergence of the self-consistent loop

In one or more embodiments, a self-consistent procedure that may be used to solve the inner loop (the iterative solution of the cluster mean field equations described above), may comprise the following operations (acts):

- Obtain (e.g. guess) an initial value for the magnetization $m_0$,
- Solve the Equation

$$H_s^\mathcal{C} = \sum_{i,j\in\mathcal{C}} J_{ij} S_i^z S_j^z + \frac{U}{4}\sum_{i\in\mathcal{C}} S_i^x + \sum_{i\in\mathcal{C}} h_i S_i^z,$$

and

- Calculate $\overline{m} = \frac{1}{N} \sum_i^N \langle S_i^z \rangle$ in the ground state obtained.

In some embodiments, iterations of the loop may be performed until a convergence criterion is reached.

The following describes simulation results in which the following two exemplary criteria were used:

In some embodiments, a first criterion may be based on the number of inner loop and outer loop, and may be narrowed by a number k (so the total number of loop iterations allowed is k × k).

In some embodiments, a second criterion may be based on the norm of the difference between Q at step 1 - 1 and Q at step 1 for the outer loop and the norm of the difference between m at step 1 - 1 and m at step 1 for the inner loop.

In some embodiments, a threshold value η may be used such that the loop may be stopped in cases where one of the two norms is lower than η. For example, the threshold value η = 0.01, which was used in the simulation described herein, may be used.

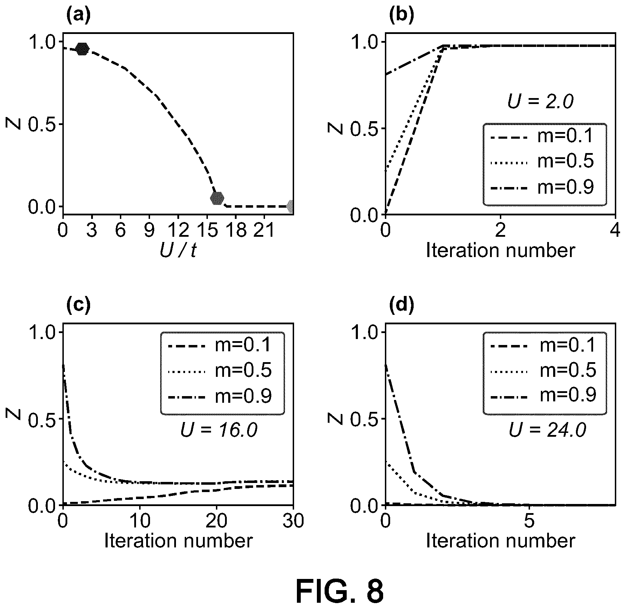

The evolution of Z as a function of a number of iterations is shown in Fig. 8(b)-(d) for an exemplary cluster of N = 6 sites, k = 10 and a threshold value η = 10⁻⁶.

Fig. 8 (b) to (d) illustrate the evolution of Z as a function of loop iterations for an exemplary 6 sites embedding.

Fig. 8(a) shows a Mott transition for an exemplary 3 × 3 cluster where three points are highlighted.

The convergence of these points to a mean field value (Fig. 8(b) U/t = 2.0, Fig. 8(c) U/t = 16.0 and Fig. 8(d) U/t = 24.0, with t being equal to 1), namely the stabilization of the values during the slave-spin mean-field procedure is shown for different exemplary initial guesses of the mean field value $\overline{m}$ (respectively 0.1, 0.5 and 0.9).

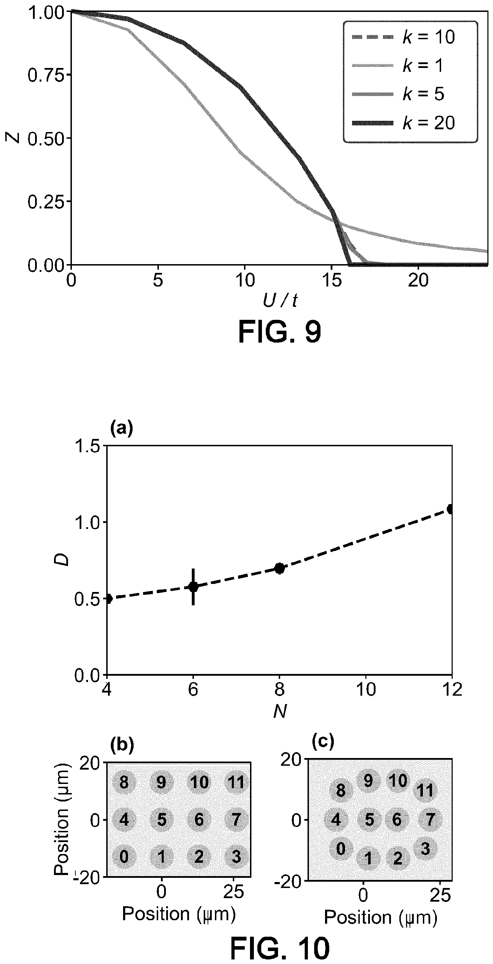

Different exemplary initial guesses for the mean field value $\overline{m}$ were tested in a simulation, and they all converged towards the same mean field value, showing the robustness of the proposed method. The convergence takes more time close to the transition value. The impact of the number k imposed is shown in Fig. 9.

Fig. 9 shows the impact of imposed number of loop iterations k, namely the number of "outer loops", i.e back-and-forths between the fermionic and the spin model, in the slave-spin mean-field scheme for a cluster of N = 4 sites. The resolution method used for the simulation was annealing, and all sources of error were neglected.

Appendix C - Solving the spin model with the Rydberg-based processor

In the following, a description of a method for solving the spin model with a Rydberg-based processor according to embodiments of the present subject disclosure and corresponding simulation results are provided.

In one or more embodiments, in order to solve the following finite spin problem Hamiltonian operator $H_s^C$ :

$$H_s \approx H_s^C = \sum_{i,j \in C} J_{ij} S_i^z S_j^z + \frac{U}{4} \sum_{i \in C} S_i^x + \sum_{i \in C} h_i S_i^z$$ , it may be advantageously considered that the Transverse-field Ising Hamiltonian operator: $$\hat{H}_{Rydberg} = \sum_{i \neq j} \frac{C_6}{|r_i - r_j|^6} \hat{n}_i \hat{n}_j + \frac{\hbar \Omega(\tau)}{2} \sum_i \hat{S}_i^x - \hbar \delta(\tau) \sum_i \hat{n}_i$$ is (naturally) implemented on an analogue quantum processor, such as for example a Rydberg-based quantum processor.

As discussed in the present subject disclosure, in some embodiments an annealing procedure may be applied with the final Hamiltonian ($\hat{H}_{Rydberg}$) as close as possible to the Hamiltonian ( $H_s^C$ ) whose ground state features the correlation functions to be computed.

In this section, an exemplary annealing procedure and the deviations from the ideal case are described in some details.

*C.1 - Optimization of the geometry*

In one or more embodiments, the Hamiltonian operator $H_s$ may display a self-consistently determined spin coupling matrix $J_{ij}$, while the Hamiltonian controlled in the experiment $\hat{H}_{Rydberg}$ may feature a van der Waals interaction term $\sum_{j \neq i} C_6 / r_{ij}^6$ .

This section explains how the geometry of the atom array (that is, the positions of the atoms) may be optimized in some embodiments so that both couplings match, in some embodiments as closely as possible.

In some embodiments, a cost function, such as for example based on the following cost function, may be minimized using any suitable optimization scheme:

$$\mathcal{D} = \sqrt{\sum_{i,j} (\frac{C_6}{|r_{i,j}|^6} + 4J_{i,j})^2} \qquad (C1)$$

In the following, embodiments of the present subject disclosure are described in the exemplary case where the above exemplary cost function $\mathcal{D}$ is used. However, a person of ordinary skill in the art would understand that the processes, apparatuses and computer programs of the present subject disclosure may be implemented based on any suitable cost function, and that such proposed processes, apparatuses and computer programs of the present subject disclosure are not limited to the use of the above cost function $\mathcal{D}$ (C1), which is provided as an example only.

In some embodiments, the conjugate gradient descent algorithm may for example be used, for example with the following initial guess for the geometry: the atoms are placed on a square lattice where the distance between nearest-neighbor atoms is:

$$r_{init} = \max_{ij}[(C_6/|4J_{ij}|)^{\frac{1}{6}}]$$

The evolution of the exemplary cost function $\mathcal{D}$ as a function of the number of sites in the cluster is illustrated on Fig. 10.

Fig. 10 (a) - (c) show an exemplary optimization of geometry for an implementation on a real quantum computer device.

Fig. 10 (a) shows the evolution of an exemplary mean value of the cost function $\mathcal{D}$ (Equation C1) according to embodiments for different cluster sizes at U = 13.1 MHz.

The error bar shows the standard deviation over all cost function $\mathcal{D}$ values encountered during loops.

Fig. 10 (b) shows an exemplary initial position of the atoms before optimization for a N = 12 sites cluster in the last outer loop iteration of the slave-spin mean-field method according to embodiments at U = 13.1 MHz.

Fig. 10 (c) shows an exemplary position of atoms after the optimization of the geometry that may be performed in some embodiments to minimize the cost function value $\mathcal{D}$.

In embodiments where the optimization of the positions does not lead to a vanishing cost function value $\mathcal{D}$, the gradient descent algorithm may be trapped in numerous local minima, leading to a poor approximation of $-4 \times J_{ij}$ by the interaction matrix element.

In addition, difficulties may arise directly from the symmetries of the initial cluster guess. For instance, in the case of a 2 × 2 cluster with nearest-neighbor distance a, the distance between next nearest neighbors is always $a/\sqrt{2}$ whereas it should be 0 for the above-described model since $J_{jj} = 0$ for the next nearest neighbors. Therefore, in most cases, the cost function value $\mathcal{D}$ may not be exactly zero.

Simulations that were made show that the impact of considering an imperfect optimization of the geometry (leading to a nonzero the cost function value D) does not however lead to significant changes, so that an imperfect optimization of the geometry may be used in one or more embodiments of the proposed method.

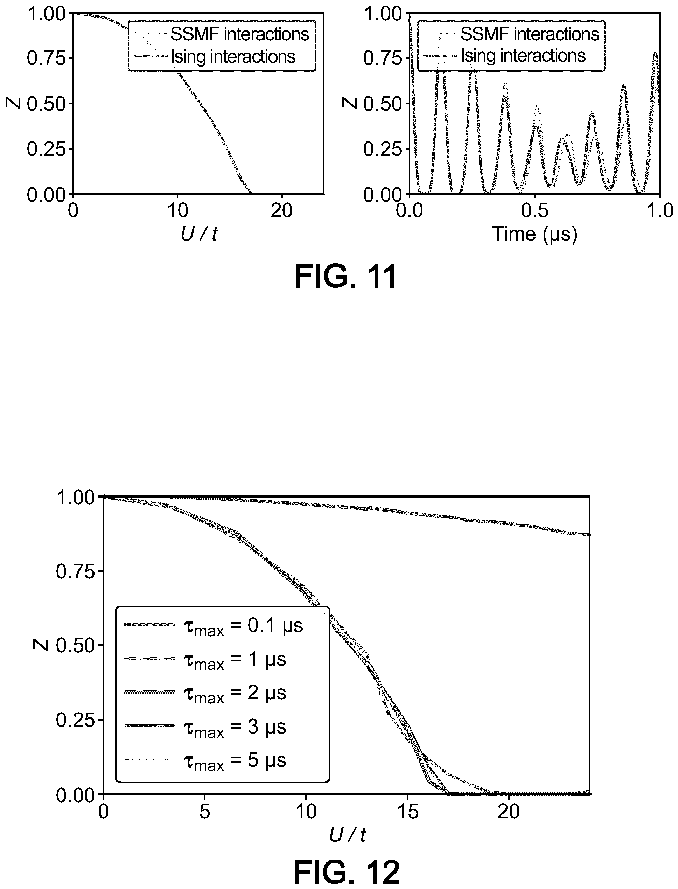

Figure 11 shows the exemplary outcome of the equilibrium (left handside graph) and out-of-equilibrium (right handside graph) computations with a "perfect geometry" (assuming the coupling is actually $J_{ij}$) and an imperfect geometry.

Fig. 11 illustrates the impact of considering a realistic geometry on a cluster of N = 4 sites. The left-hand side of the figure shows a comparison of Z values between method with the real matrix J and the optimized one for 4 sites, for an imperfect (non-zero cost function D) optimization of the geometry (Ising interactions) versus a perfect (zero cost function D) optimization of the geometry (Strong Spinning Magnetic Force (SSMF) interactions).

The right-hand side shows Z dynamics after a quench $U_f$ = 13 MHz with the same comparison.

For the Mott transition, we observe negligible differences for an exemplary N = 4 cluster. For the dynamical behavior, we do observe a change in amplitude but the frequency remains the same as for the slave-spin mean-field interactions.

Figures 10(b) and 10(c) respectively show exemplary initial and optimized position for N = 12 cluster sites that illustrates the outcome of the optimization procedure according to embodiments of the present subject disclosure.

As shown on Fig. 10, the final pattern is slightly distorted compared to the translation-invariant initial pattern. This is due to the fact that the couplings at the edges of the cluster differ from the ones in the "bulk" of the cluster to account for the cluster's environment. As the cluster size grows, these edge effects have decreasing influence, and the optimization becomes easier. It may therefore be concluded that the geometry optimization proposed according to embodiments of the present subject disclosure yields reasonably faithful interactions.

## C.2 - Details of the annealing schedule

Once the geometry is found according to embodiments of the present subject disclosure, the atoms of the quantum processor used for implementing the proposed method according to embodiments of the present subject disclosure may be prepared in the state $|\Psi_{start}\rangle = |g\rangle^{\otimes N}$. The following Hamiltonian is the one applied at $\tau = 0$

$$H_{start} = \sum_{i \neq j} \frac{C_6}{|r_i - r_j|^6} \hat{n}_i \hat{n}_j - \hbar \delta_{start} \sum_i n_i \qquad (C2)$$

where $\frac{\delta_{start}}{2\pi} = -5$ MHz so that $|\Psi_{start}\rangle$ is the most excited state in Equation C2.

In some embodiments, the Rabi frequency ($\Omega(t)$)and the detuning ($\delta(t)$), addressing globally the array, may then be varied over a total annealing time duration $\tau_{max}$ to reach the Hamiltonian -

$$H_s^{\mathcal{C}}.$$

According to embodiments of the proposed method, the Rabi frequency may start at 0 MHz and may be driven linearly to U/2 ($\hbar\Omega(\tau_{max}) = U/2$): For example, in some embodiments, $\Omega(t)$ may be approximated as a linear function of time starting from 0 *MHz* to a final value *U/2.*

Similarly, in some embodiments, the de-tunings may all be prepared at a value $\delta_{start}$ and may be driven separately to values:

$$\hbar \delta_i(\tau_{max}) = \sum_{j \neq i} C_6 / r_{ij}^6 + 4\bar{j}\overline{m}z_i$$

Fig. 12 shows an exemplary effect of the total annealing time $\tau_{max}$ on the outcome of the simulation: The longer $\tau_{max}$, the better the results of the simulation, and with $\tau_{max} = 3 \mu s$, the results can be considered as being essentially stable. As a consequence, it can be concluded that increasing the value of $\tau_{max}$ beyond 3 $\mu s$ would not significantly improve the results. As a consequence, the parameter $\tau_{max}$ can be determined according to the above procedure.

All other sources of noise (dephasing, readout, shot noise, etc) may in some embodiments be neglected. Beyond the value $\tau_{max} = 3\mu$ s, the influence of the parameter $\tau_{max}$ may be considered in some embodiments negligible. In our simulations, we choose $\tau_{max} = 4\mu s$ to ensure a good convergence.

In one or more embodiments, the value of the Rabi term may be quenched in order to study the dynamics in the Hubbard model. The quench procedure can be used in some embodiments to investigate the material in a dynamic mode, that is, in a non-equilibrium phase.

In some embodiments, owing to the finite response time of the optical modulators used in an experimental setting, the quench may not be instantaneous.

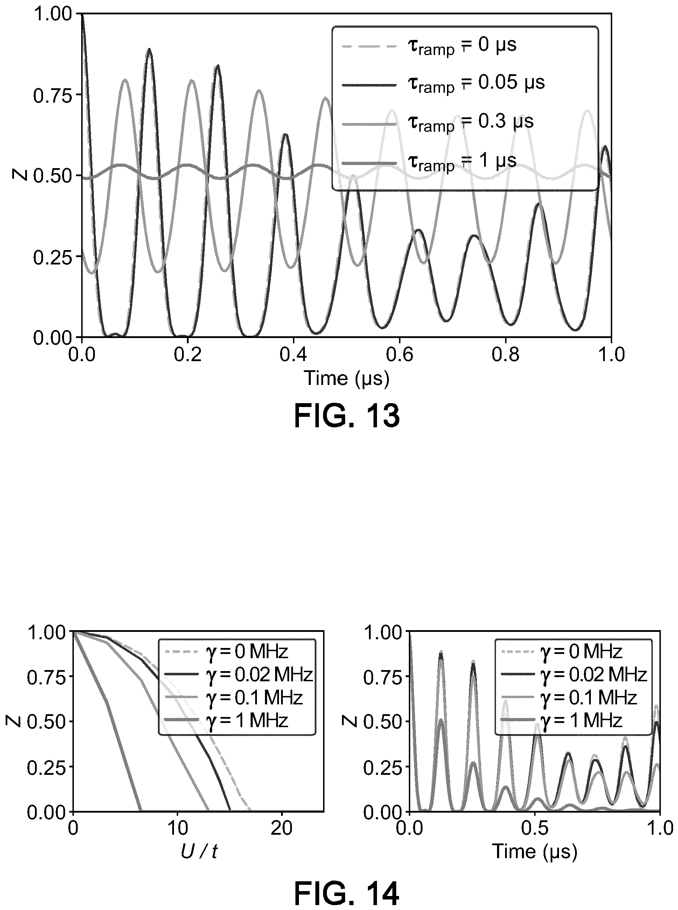

Fig. 13 illustrates an exemplary impact of a switch-on time $\tau_{ramp}$ in the quench dynamic for a cluster of N = 4 sites, for an exemplary $U_f$ = 13MHz ($U_f$ designating the value set for the parameter *U* (*f* as final), which corresponds to $\Omega(\tau_{max})$).

All other sources of error may in some embodiments be neglected.

Fig. 13 illustrates the impact of the finite switch-on time $\tau_{ramp}$ of the optical modulators used for the simulation on the Rabi frequency and the detuning.

As shown on Fig. 13, the finite switch-on time $\tau_{ramp}$does impact the frequency of the signal for $\tau_{ramp} \geq 0.3\mu s$. Therefore, in embodiments where a modulator whose technical specifications indicates $\tau_{ramp} \approx 0.05\mu s$ is used, the effects of the finite switch-on time $\tau_{ramp}$ on the Rabi frequency and the detuning can be considered negligible.

## C.3 - Experimental imperfections

The proposed method described above may advantageously be implemented on existing Rydberg processors.

To evaluate the effects of noise and experimental limitations on the results, we include them in the simulation.

The following provides a description of methods that may be used in some embodiments to emulate the noise and how they may be implemented in software. All numerical simulations, and the State Preparation and Measurement (SPAM) error, may be performed with any suitable software libraries.

*Dephasing noise*

Decoherence during the annealing procedure is described via the Lindblad master equation:

$$\frac{d\rho}{d\tau} = -i[H(\tau), \rho] - \frac{1}{2}\sum_{i=1}^{N} \gamma_i \left[\{L_i^\dagger L_i, \rho\} - 2L_i \rho L_i^\dagger\right] \qquad (C3)$$

where $\rho$ is the density matrix of the system and $H(\tau)$ is the resource Hamiltonian at a time $\tau$ during the annealing. The jump operators $L_i$ corresponding to dephasing are equal to $n_i$. For the sake of simplicity, we use a single dephasing parameter $\gamma_j = \gamma$.

The effect of the dephasing amplitude $\gamma$ is shown for various dephasing parameter $\gamma$ values in Fig. 14. For the quench dynamics, the dephasing damps the oscillations but does not change the frequency. We can see this behaviour on the position of the Mott transition, which is towards small values of U/t for large $\gamma$.

Figure 14 illustrates an exemplary effect of dephasing noise on the result for a cluster of $N$ = 4 sites: the larger $\gamma$, the more deformed (damped) the curve The left handside of the figure illustrates an exemplary impact on the Mott phase transition. All other sources of noise are neglected.

The right handside of the figure illustrates an exemplary impact on the quench dynamic for $U_f$ = 13MHz the larger $\gamma$, the more deformed (damped) the curve

*Sampling and measurement error*

In one or more embodiments, the sampling of states may be simulated as would be done on an analogue processor (e.g. a Rydberg processor) (shot-noise) by picking randomly $N_s$ times a bitstring with a probability equal to the probability to measure this bitstring in the z-basis: In some embodiments, in order to determine the correlation < *S S* >, $N_s$ samples may be measured, and < *S S* > may be approximated by a mean over the $N_s$ samples.

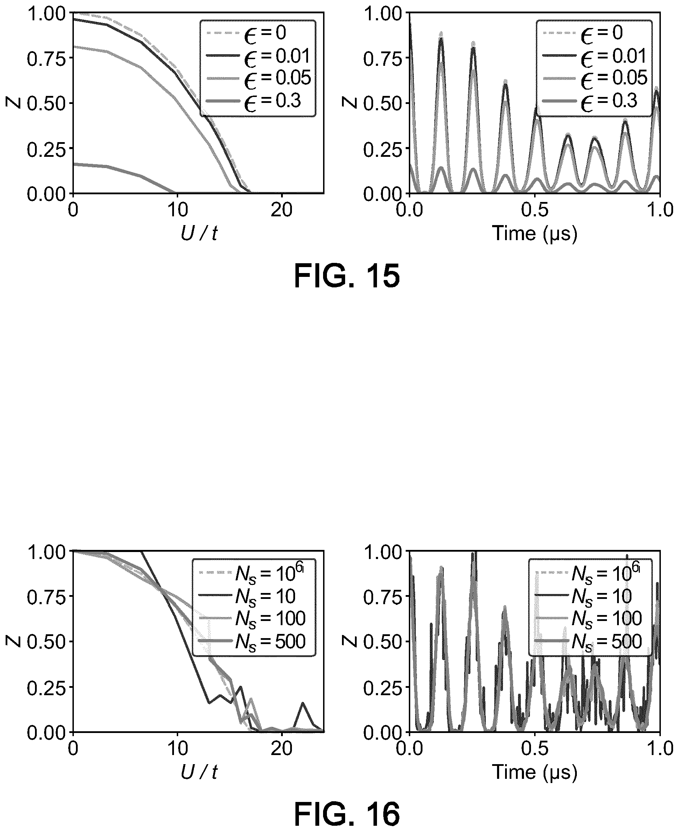

The impact of such a sampling procedure is shown in Fig. 15 at equilibrium and out of equilibrium: it becomes negligible as soon as $N_s$ is chosen beyond a certain threshold (e.g. chosen equal 100).

Figure 16 shows an exemplary impact of different sampling rates *$N_s$* on measured states for a cluster of $N$ = 4 sites.

The left handside of the figure shows an exemplary impact of different sampling rates $N_s$ on measured states for a cluster of $N$ = 4 sites at an equilibrium phase.

The right handside of the figure shows an exemplary impact of different sampling rates $N_s$ on measured states for a cluster of $N$ = 4 sites at an out of equilibrium phase ($U_f$ = 13 MHz). All other sources of error are neglected.

In some embodiments, the readout error may be modelled by a probability of error $\varepsilon$ of detecting an atom in a state $|r\rangle$ instead of its real state $|g\rangle$ and $\varepsilon'$ of not detecting an excited atom. In some embodiments, the probabilities of error values may be chosen as $\varepsilon = \varepsilon' = 3\%$ for both values. The impact of the errors for $\varepsilon = \varepsilon'$ is shown in Fig. 15. Until $\varepsilon \approx 5\%$, the behavior of the system remains the same.

Figure 15 illustrates an exemplary impact of measurement error for a cluster of $N$ = 4 sites. The left handside of the figure shows the effect of $\varepsilon = \varepsilon'$ at an equilibrium phase for $U_f$ = 13MHz.

The right handside of the figure shows the effect of $\varepsilon = \varepsilon'$ at an out of equilibrium phase for $U_f$ = 13MHz. The number of shots considered for each measurement is $10^6$. All other sources of noise are neglected.

**Claims**

1. A computer-implemented method for determining a quantum phase parameter characterizing a property of a quantum phase of a solid-state material sample, the method comprising:

   Obtaining an estimate of a Hamiltonian operator *H* of a lattice model of the solid-state material sample;
   Mapping the estimate of the Hamiltonian operator *H* onto a combination of a plurality of Hamiltonian operators comprising a Hamiltonian operator describing a longitudinal spin interaction and a transverse field;
   Computing, using a quantum processor, an estimate of a property of the Hamiltonian operator describing a longitudinal spin interaction and a transverse field; and

Computing an estimate of the quantum phase parameter as a property of the Hamiltonian operator *H,* based on the estimate of the property of the Hamiltonian operator describing the longitudinal spin interaction and the transverse field.

2. The method according to claim 1, wherein the plurality of Hamiltonian operators further comprises a free fermionic Hamiltonian operator with renormalized hopping, the method further comprising: computing, using a non-quantum processor, an estimate of a property of the free fermionic Hamiltonian operator with renormalized hopping, wherein the estimate of the quantum phase parameter is further based on the estimate of the property of the free fermionic Hamiltonian operator.

3. The method according to any of the previous claims, wherein the Hamiltonian operator describing a longitudinal spin interaction and a transverse field comprises a transverse field Ising Hamiltonian operator.

4. The method according to any of the previous claims, wherein the Hamiltonian operator describing a longitudinal spin interaction and a transverse field comprises a combination of a longitudinal spin interaction term and a transverse field term.

5. The method according to any of the preceding claims, wherein the property relates to a quasiparticle weight of the solid-state material sample.

6. The method according to any of the preceding claims, wherein the parameter characterizes the behaviour of the solid-state material sample in an equilibrium state of the solid-state material sample.

7. The method according to any of the preceding claims, wherein the parameter characterizes the behaviour of the solid-state material sample in an out of equilibrium state of the solid-state material sample.

8. The method according to any of the previous claims, wherein obtaining the estimate of the Hamiltonian operator *H* of the lattice model of the solid-state material sample comprises:

   Obtaining an estimate of a hopping parameter t for the solid-state material sample;
   Obtaining an estimate of an on-site interaction parameter *U* for the solid-state material sample.

9. The method according to claim 8, wherein the obtaining the estimate of the hopping parameter *t* for the solid-state material sample comprises: measuring the estimate of hopping parameter *t* on the solid-state material sample.

10. The method according to any of claims 8 and 9, wherein the obtaining the estimate of the on-site interaction parameter *U* for the solid-state material sample comprises: measuring the estimate of the on-site interaction parameter *U* on the solid-state material sample.

11. The method according to any of the preceding claims, wherein the model is a Fermi-Hubbard model describing the material as a lattice with interacting electrons.

12. The method according to any of the preceding claims, wherein the quantum processor is an analogue quantum processor.

13. The method according to any of the preceding claims, further comprising: Solving the Hamiltonian operator of the Fermi-Hubbard model by performing one or more iterations of a model solving loop, an iteration of the model solving loop comprising:

   - Computing, using the non-quantum processor, a correlation function $\langle f^+f\rangle_{nn}$ of the transverse field Ising Hamiltonian operator based on an initial value of a renormalized hopping parameter $Q$;
   - Using the correlation function $\langle f^+f\rangle_{nn}$ of the transverse field Ising Hamiltonian operator to compute, using the quantum processor, a correlation function $\langle S^z S^z\rangle_{nn}$ of the free fermionic Hamiltonian operator;
   - Compute a value of the renormalized hopping parameter $Q$ based on the correlation function $\langle S^z S^z\rangle_{nn}$.

14. A quantum computer apparatus, the apparatus comprising a quantum processor and a memory operatively coupled to the processor, wherein the apparatus is configured to compute, using the quantum processor, an estimate of a property of a Hamiltonian operator describing a longitudinal spin interaction and a transverse field as part of performing a method according to any of claims 1 to 13.

15. A computer program product comprising computer program code embodied in a computer readable medium, said computer program code comprising instructions to, when provided to a quantum computer system and executed, cause said computer to compute, using a quantum processor of the quantum computer system, an estimate of a property of a Hamiltonian operator describing a longitudinal spin interaction and a transverse field as part of performing a method according to any of claims 1 to 13.

FIG. 1

| Obtaining an estimate of a Hamiltonian operator *H* of a crystal lattice model of the crystal sample |
|---|

21

| Mapping the estimate of the Hamiltonian operator *H* onto a combination of a plurality of Hamiltonian operators comprising a Hamiltonian operator describing a longitudinal spin interaction and a transverse field |
|---|

22

| Computing, using a quantum processor, an estimate of a property of the Hamiltonian operator describing a longitudinal spin interaction and a transverse field |
|---|

23

| Computing an estimate of the quantum phase parameter as a property of the Hamiltonian operator H, based on the estimate of the property of the Hamiltonian operator describing the longitudinal spin interaction and the transverse field |
|---|

24

FIG. 2

$$-t$$
$$U$$
13

$$(f^\dagger f)_{nn}$$
$$J = -t(f^\dagger f)_{nn}$$
13b

13a

$$Q = -t(S^z S^z)_{nn}$$
$$(S^z S^z)_{nn}$$
$$-U/4$$

FIG. 3

FIG. 4

**FIG. 5**

FIG. 6

FIG. 7

FIG. 8

FIG. 9

FIG. 10

**FIG. 11**

**FIG. 12**

FIG. 13

FIG. 14

FIG. 15

FIG. 16

Europäisches
Patentamt

European
Patent Office

Office européen
des brevets

# EUROPEAN SEARCH REPORT

**Application Number**

EP 23 30 7166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANTOINE BROWAEYS ET AL: "Many-Body Physics with Individually-Controlled Rydberg Atoms", ARXIV.ORG, CORNELL UNIVERSITY LIBRARY, 201 OLIN LIBRARY CORNELL UNIVERSITY ITHACA, NY 14853, 18 February 2020 (2020-02-18), XP081602099, DOI: 10.1038/S41567-019-0733-Z * abstract * * sectiosn I, IV, V * ----- | 1-15 | INV. G06N10/20 G06N10/40 G06N10/60 G16C10/00 |
| X | JONATHAN SIMON ET AL: "Quantum Simulation of Antiferromagnetic Spin Chains in an Optical Lattice", ARXIV.ORG, CORNELL UNIVERSITY LIBRARY, 201 OLIN LIBRARY CORNELL UNIVERSITY ITHACA, NY 14853, 7 March 2011 (2011-03-07), XP080542771, DOI: 10.1038/NATURE09994 * abstract * * page 1 - page 4 * * page 7 * * figure 1 * ----- | 1-15 | |
| X | ZHANG J ET AL: "Observation of a Many-Body Dynamical Phase Transition with a 53-Qubit Quantum Simulator", ARXIV.ORG, CORNELL UNIVERSITY LIBRARY, 201 OLIN LIBRARY CORNELL UNIVERSITY ITHACA, NY 14853, 3 August 2017 (2017-08-03), XP080951234, DOI: 10.1038/NATURE24654 * abstract * * page 1 - page 2 * * Appendices C, D * ----- -/-- | 1-15 | |

TECHNICAL FIELDS
SEARCHED       (IPC)

G06N
G16C

The present search report has been drawn up for all claims

| Place of search | Date of completion of the search | Examiner |
|---|---|---|
| The Hague | 26 April 2024 | Papadakis, Georgios |

EPO FORM 1503 03.82 (P04C01)

**page 1 of 2**

**EUROPEAN SEARCH REPORT**

Europäisches Patentamt
European Patent Office
Office européen des brevets

**Application Number**

EP 23 30 7166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARCO SCHIRO' ET AL: "Quantum Quenches in the Hubbard Model: Time Dependent Mean Field Theory and The Role of Quantum Fluctuations", ARXIV.ORG, CORNELL UNIVERSITY LIBRARY, 201 OLIN LIBRARY CORNELL UNIVERSITY ITHACA, NY 14853, 8 February 2011 (2011-02-08), XP080497902, DOI: 10.1103/PHYSREVB.83.165105 * abstract * * section IV * ----- | 1-15 | |

TECHNICAL FIELDS SEARCHED (IPC)

The present search report has been drawn up for all claims

| Place of search | Date of completion of the search | Examiner |
|---|---|---|
| The Hague | 26 April 2024 | Papadakis, Georgios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2